# EUROPEAN PATENT SPECIFICATION

(11) **EP 2 782 931 B1**
(45) Date of publication and mention of the grant of the patent: **14.12.2016**
(21) Application number: 12788221.5
(22) Date of filing: 21.11.2012
(51) Int. Cl.: C07K 16/00, C07K 16/28, C12N 5/0781

(54) **CD40L EXPRESSING MAMMALIAN CELLS AND THEIR USE**
SÄUGERZELLEN ZUR CD40L-EXPRESSION UND IHRE VERWENDUNG
EXPRESSION AU CD40L DES CELLULES DE MAMMIFÈRES ET UTILISATION

(30) Priority: 23.11.2011 EP 11190341
(43) Date of publication of application: 01.10.2014
(73) Proprietor: F.Hoffmann-La Roche AG, 4070 Basel (CH)
(72) Inventor: ENDL, Josef, 82362 Weilheim (DE); FISCHER, Jens, 82362 Weilheim (DE); KERN, Peter, 82377 Penzberg (DE); OFFNER, Sonja, 82377 Penzberg (DE); PLATZER, Josef, 82538 Geretsried (DE); SEEBER, Stefan, 82404 Sindelsdorf (DE)
(74) Representative: Burger, Alexander
(86) International application number: PCT/EP2012/073232
(87) International publication number: WO 2013/076139

(56) References cited:
- WO-A1-2011/052545
- WO-A2-2008/085878
- WO-A2-2009/105150
- AMANNA I J ET AL: "Quantitation of rare memory B cell populations by two independent and complementary approaches", JOURNAL OF IMMUNOLOGICAL METHODS, ELSEVIER SCIENCE PUBLISHERS B.V.,AMSTERDAM, NL, vol. 317, no. 1-2, 20 December 2006 (2006-12-20), pages 175-185, XP025158255, ISSN: 0022-1759, DOI: 10.1016/J.JIM.2006.09.005 [retrieved on 2006-12-20]

### SEQUENCE LISTING

<110> F. Hoffmann-La Roche AG
<120> CD40L expressing mammalian cells and their use
<130> 30716 WO
<150> EP11190341.5
   <151> 2011-11-23
<160> 3
<170> PatentIn version 3.5
<210> 1
   <211> 261
   <212> PRT
   <213> Oryctolagus cuniculus
<400> 1
   Met Ile Glu Thr Tyr Ser Gln Pro Thr Pro Arg Ser Val Ala Thr Gly
   1               5                   10                  15
   Pro Ser Val Ser Met Lys Ile Phe Met Tyr Leu Leu Thr Val Phe Leu
               20                  25                  30
   Ile Thr Gln Met Ile Gly Ser Ala Leu Phe Ala Val Tyr Leu His Arg
           35                  40                  45
   Arg Leu Asp Lys Ile Glu Asp Glu Arg Asn Leu His Glu Asp Phe Val
       50                  55                  60
   Phe Met Lys Thr Ile Gln Arg Cys Asn Lys Gly Glu Gly Ser Leu Ser
   65                  70                  75                  80
   Leu Leu Asn Cys Lys Glu Ile Arg Ser Gln Phe Glu Gly Phe Val Lys
                   85                  90                  95
   Asp Ile Met Leu Asn Lys Glu Glu Pro Lys Lys Glu Ile Asn Phe Glu
               100                 105                 110
   Met Gln Lys Gly Asp Gln Asp Pro Gln Ile Ala Ala His Leu Ile Ser
           115                 120                 125
   Glu Ala Ser Ser Lys Ser Ser Ser Val Leu Gln Trp Ala Lys Lys Gly
       130                 135                 140
   Tyr Tyr Thr Met Ser Asn Thr Leu Val Thr Leu Glu Asn Gly Lys Gln
   145                 150                 155                 160
   Leu Lys Val Lys Arg Gln Gly Phe Tyr Tyr Ile Tyr Ala Gln Val Thr
                   165                 170                 175
   Phe Cys Ser Asn Gln Glu Pro Ser Ser Gln Ala Pro Phe Ile Ala Ser
               180                 185                 190
   Leu Cys Leu Lys Ser Ser Gly Gly Ser Glu Arg Ile Leu Leu Arg Ala
           195                 200                 205
   Ala Asn Ala Arg Ser Ser Ser Lys Thr Cys Glu Gln Gln Ser Ile His
       210                 215                 220
   Leu Gly Gly Val Phe Glu Leu Gln Ala Asp Ala Ser Val Phe Val Asn
   225                 230                 235                 240
   Val Thr Asp Ala Ser Gln Val Asn His Gly Thr Gly Phe Thr Ser Phe
                   245                 250                 255
   Gly Leu Leu Lys Leu
               260
<210> 2
   <211> 261
   <212> PRT
   <213> Homo sapiens
<400> 2
<210> 3
   <211> 260
   <212> PRT
   <213> Mus musculus
<400> 3
   Met Ile Glu Thr Tyr Ser Gln Pro Ser Pro Arg Ser Val Ala Thr Gly
   1               5                   10                  15
   Leu Pro Ala Ser Met Lys Ile Phe Met Tyr Leu Leu Thr Val Phe Leu
               20                  25                  30
   Ile Thr Gln Met Ile Gly Ser Val Leu Phe Ala Val Tyr Leu His Arg
           35                  40                  45
   Arg Leu Asp Lys Val Glu Glu Glu Val Asn Leu His Glu Asp Phe Val
       50                  55                  60
   Phe Ile Lys Lys Leu Lys Arg Cys Asn Lys Gly Glu Gly Ser Leu Ser
   65                  70                  75                  80
   Leu Leu Asn Cys Glu Glu Met Arg Arg Gln Phe Glu Asp Leu Val Lys
                   85                  90                  95
   Asp Ile Thr Leu Asn Lys Glu Glu Lys Lys Glu Asn Ser Phe Glu Met
               100                 105                 110
   Gln Arg Gly Asp Glu Asp Pro Gln Ile Ala Ala His Val Val Ser Glu
           115                 120                 125
   Ala Asn Ser Asn Ala Ala Ser Val Leu Gln Trp Ala Lys Lys Gly Tyr
       130                 135                 140
   Tyr Thr Met Lys Ser Asn Leu Val Met Leu Glu Asn Gly Lys Gln Leu
   145                 150                 155                 160
   Thr Val Lys Arg Glu Gly Leu Tyr Tyr Val Tyr Thr Gln Val Thr Phe
                   165                 170                 175
   Cys Ser Asn Arg Glu Pro Ser Ser Gln Arg Pro Phe Ile Val Gly Leu
               180                 185                 190
   Trp Leu Lys Pro Ser Ser Gly Ser Glu Arg Ile Leu Leu Lys Ala Ala
           195                 200                 205
   Asn Thr His Ser Ser Ser Gln Leu Cys Glu Gln Gln Ser Val His Leu
       210                 215                 220
   Gly Gly Val Phe Glu Leu Gln Ala Gly Ala Ser Val Phe Val Asn Val
   225                 230                 235                 240
   Thr Glu Ala Ser Gln Val Ile His Arg Val Gly Phe Ser Ser Phe Gly
                   245                 250                 255
   Leu Leu Lys Leu
               260

## Description

The current patent application is in the field of B-cell cultivation. Herein is reported a culture system for stimulation and expansion of rabbit and human derived antibody-secreting cells, such as activated B-cells, plasmablasts and B-cells isolated from immunized rabbits or humans after survival of an illness in the presence of rabbit or human CD40L-expressing feeder cells.

### Background of the Invention

It has been reported that isolated B-cells, plasmablasts and plasma cells from different species were difficult to culture in vitro. In practice this is normally overcome by the generation of immortal B-cell lines by fusing primary B-cells to a hybridoma partner or immortalization. For rabbits also a plasmacytoma fusion partner was developed (Spieker-Polet, H., et. al., Proc. Natl. Acad. Sci. USA 92 (1995) 9348-9352, but the hybridoma technique does not work well in rabbits. For sustained survival of rabbit B-cells in vitro it is necessary to provide essential activation stimuli and survival factors. A variety of compounds have been described such as IL-2, IL-4, IL-10 and others (see e.g. Zubler, R., et al., Eur. J. Immunol. 14 (1984) 357-363, and J. Exp. Med. 160 (1984) 1170-1183).

Physiologically, B-cells are activated upon encounter with a specific antigen plus additional activation e.g. via the CD40/CD40L pathway (reviewed in Néron, S., et al., Arch. Immunol. Ther. Exp. 59 (2011) 25-40) and/or via cytokines and/or growth factors derived from the natural context, e.g. by Dendritic cells or upon T-cell help.

In in vitro systems the CD40/CD40L interaction can be mimicked by co-culture with EL4-B5 thymoma cells or the addition of soluble or immobilized anti-CD40 antibodies or CD40L. The sole systems lack sufficient potency and are only described in combination with further required stimuli using either different feeder mixes (e.g. Zubler mix; IL-4, IL-10, etc.) (Zubler, R., supra) or thymocyte supernatant (TSN).

Weitkamp, J-H., et al., (J. Immunol. Meth. 275 (2003) 223-237) report the generation of recombinant human monoclonal antibodies to rotavirus from single antigen-specific B-cells selected with fluorescent virus-like particles. A method of producing a plurality of isolated antibodies to a plurality of cognate antigens is reported in US 2006/0051348. In WO 2008/144763 and WO 2008/045140 antibodies to IL-6 and uses thereof and a culture method for obtaining a clonal population of antigen-specific B-cells are reported, respectively. A culture method for obtaining a clonal population of antigen-specific B-cells is reported in US 2007/0269868. Masri, et al. (in Mol. Immunol. 44 (2007) 2101-2106) report the cloning and expression in E.coli of a functional Fab fragment obtained from single human lymphocyte against anthrax toxin. A method for preparing immunoglobulin libraries is reported in WO 2007/031550.

In WO 2009/10515 a method of making hybrid cells that express useful antibodies is reported. High affinity antibodies that neutralize Staphylococcus enterotoxin B are reported in WO 2008/085878. Amanna, I.J., and Slifka, M.K., report the quantitation of rare memory B cell populations by two independent and complementary approaches (J. Immunol. Meth. 317 (2006) 175-185). In WO 2011/052545 a method for producing antigen-specific B cell population is reported.

### Summary of the Invention

Herein is reported a new method for the rapid, efficient and reproducible generation of rabbit (or human) antibodies starting from rabbit (or human) B-cells and comprising at least one cultivation step.

Herein is further reported a feeder mix that can be used in combination with rabbit CD40L-expressing mammalian cells providing a highly efficient rabbit B-cell stimulation and cultivation system.

Herein is further reported a feeder mix that can be used in combination with human CD40L-expressing mammalian cells providing a highly efficient human B-cell stimulation and cultivation system.

One aspect as reported herein is a method for co-cultivating rabbit B-cells and rabbit CD40L expressing CHO or BHK cells in the presence of/together with IL-2 and IL-21.

Reported herein is a method for co-cultivating human B-cells and human CD40L expressing mammalian cells.

Reported herein is a method for co-cultivating human B-cells and human CD40L expressing mammalian cells in the presence of/together with IL-2 and/or IL-21 and/or IL-6.

In one embodiment the human CD40L expressing mammalian cell is a BHK or CHO cell.

In one embodiment the co-cultivating is in the presence of IL-2 and IL-21.

In one embodiment the co-cultivating is in the presence of IL-2 and IL-21 and IL-6.

In one embodiment the interleukins used in the co-cultivating are all human interleukins.

One aspect as reported herein is a method for producing a rabbit antibody comprising the step of cultivating one or more antibody secreting rabbit B-cells and rabbit CD40L expressing CHO or BHK cells in the presence of/together with IL-2 and IL-21 and recovering the antibody from the cultivation supernatant, thereby producing the rabbit antibody.

In one embodiment the IL-2 and IL-21 are added at the start of the cultivation.

In one embodiment the IL-2 and IL-21 are added solely at the start of the cultivation.

In one embodiment the rabbit B-cell is a naive or non-mature rabbit B-cell.

In one embodiment the B-cell is an IgG positive B-cell (IgG⁺). IgG positive B-cells present the cell surface marker IgG which can be detected and labeled.

Reported herein is a method for producing a human antibody comprising the step of cultivating an antibody secreting human B-cell and human CD40L expressing mammalian cells and recovering the antibody from the cultivation supernatant, thereby producing the human antibody.

In one embodiment the human CD40L expressing mammalian cell is a BHK or CHO cell.

Reported herein is a method for producing a human antibody comprising the step of cultivating one or more antibody secreting human B-cells and human CD40L expressing mammalian cells in the presence of/together with IL-2 and/or IL-21 and/or IL-6 and recovering the antibody from the cultivation supernatant, thereby producing the human antibody.

In one embodiment the cultivating is in the presence of IL-2 and IL-21.

In one embodiment the cultivating is in the presence of IL-2 and IL-21 and IL-6.

In one embodiment the interleukins used in the cultivating are all human interleukins.

In one embodiment the human CD40L expressing mammalian cell is a BHK or CHO cell.

In one embodiment the interleukins are added at the start of the cultivation.

In one embodiment the interleukins are added solely at the start of the cultivation.

In one embodiment the human B-cell is a mature human B-cell.

In one embodiment the B-cell is an IgG positive B-cell (IgG⁺). IgG positive B-cells present the cell surface marker IgG which can be detected and labeled.

Reported herein is a kit comprising rabbit CD40L expressing CHO cells and IL-2 and IL-21.

Reported herein is a kit comprising human CD40L expressing BHK cells and IL-2 and/or IL-21 and/or IL-6.

In one embodiment the kit comprises IL-2 and IL-21.

In one embodiment the kit comprises IL-2 and IL-21 and IL-6.

In one embodiment the interleukins are all human interleukins.

Reported herein is a method for the co-cultivation of B-cells comprising the co-cultivation of B-cells and CD40L expressing mammalian cells in the presence of IL-2 or IL-21.

Reported herein is a method for the co-cultivation of B-cells comprising the co-cultivation of B-cells and CD40L expressing mammalian cells in the presence of IL-2 and IL-21.

Reported herein is a method for the co-cultivation of rabbit B-cells comprising the co-cultivation of rabbit B-cells and rabbit CD40L expressing mammalian cells in the presence of IL-2 and IL-21.

In one embodiment the mammalian cell is a CHO cell or a BHK cell.

Reported herein is a method for the co-cultivation of B-cells comprising the co-cultivation of B-cells and CD40L expressing mammalian cells in the presence of IL-2 and/or IL-21 and/or IL-6.

In one embodiment the co-cultivating is in the presence of IL-2 and IL-21.

In one embodiment the co-cultivating is in the presence of IL-2 and IL-21 and IL-6.

Reported herein is a method for the co-cultivation of human B-cells comprising the co-cultivation of human B-cells and human CD40L expressing mammalian cells in the presence of IL-2 and/or IL-21 and/or IL-6.

In one embodiment the mammalian cell is a BHK cell or a CHO cell.

In one embodiment the co-cultivating is in the presence of IL-2 and IL-21.

In one embodiment the co-cultivating is in the presence of IL-2 and IL-21 and IL-6.

In one embodiment the interleukins used in the co-cultivating are all human interleukins.

Reported herein is a method for cultivating B-cells secreting T-cell-inhibiting compounds comprising the co-cultivation of the B-cells and CD40L expressing mammalian cells.

Reported herein is a method for the cultivation of antibody secreting B-cells comprising in the following order
i) a first co-cultivation of the B-cell and a CD40L expressing mammalian cell in the presence of a mitogenic stimulant, and
ii) a subsequent second co-cultivation of the B-cell and the CD40L expressing mammalian cell in the presence of an antibody production stimulant.

In one embodiment the first and second co-cultivation are performed in the same cultivation vessel.

In one embodiment the method for the cultivation of antibody secreting B-cells comprises a co-cultivation of the B-cell and a CD40L expressing mammalian cell whereby at first a mitogenic stimulant is added to the cultivation and thereafter an antibody production stimulant is added to the cultivation.

In one embodiment the antibody production stimulant is added at least one hour after the mitogenic stimulant is added. In one embodiment the antibody production stimulant is added one to three days after the mitogenic stimulant is added to the cultivation.

In one embodiment the mitogenic stimulant is selected from the group comprising CD40- and CD40L-interacting compounds, ICOS- and ICOS-L-interacting compounds, APRIL, BAFF, CR2, CXCL9, CXCL12 (SDF-1), CXCL13, CXCL16, Flt-3L, Interleukin-1 (α/β), Interleukin-2, Interleukin-3, Interleukin-4, Interleukin-5, Interleukin-7, Interleukin-10, Interleukin-14, Interleukin-21, SAP (signaling lymphocyte activation molecule [SLAM] associated protein), Staphylococcus A strain Cowan 1 particles (SAC; heat-killed, formalin-fixed), TLR Ligands such as LPS, different CpG ODNs or Resiquimod (R-848), TSLP, Tumor necrosis factor (TNF) alpha, type I Interferons (e.g. IFN α/β), and type II interferon (e.g. IFNγ). B-cell activation might also be induced via anti-IgG, anti-CD20, and/or anti-CD27 antibodies.

In one embodiment the antibody production stimulant is selected from the group comprising CD40- and CD40L-interacting compounds, ICOS- and ICOS-L-interacting compounds, APRIL, BAFF, CR2, CXCL9, CXCL12 (SDF-1), CXCL13, CXCL16, Flt-3L, Interleukin-1 (α/β), Interleukin-2, Interleukin-3, Interleukin-4, Interleukin-5, Interleukin-6, Interleukin-9, Interleukin-10, Interleukin-13, Interleukin-21, Interleukin-33, SAP (signaling lymphocyte activation molecule [SLAM] associated protein), Staphylococcus A strain Cowan 1 particles (SAC; heat-killed, formalin-fixed), TLR Ligands such as LPS, different CpG ODNs or Resiquimod (R-848), TSLP, Tumor necrosis factor (TNF) alpha, type I Interferons (e.g. IFN α/β), and type II interferon (e.g. IFNγ). Further B-cell stimulation might also be induced via anti-IgG, anti-CD20, and/or anti-CD27antibodies.

In one embodiment the mitogenic stimulant is added at the beginning of the first co-cultivation.

In one embodiment the mitogenic stimulant is added 1, 2, 3, 4, 5, 6, 7, 8, 9, 10, 11, 12, 13, or 14 days after the beginning of the first co-cultivation.

In one embodiment the antibody production stimulant is added 1, 2, 3, 4, 5, 6, 7, 8, 9, 10, 11, 12, 13, or 14 days after the addition of the mitogenic stimulant.

In one embodiment each of the co-cultivations is performed for 5 to 14 days.

In one embodiment the cultivating is for a total period of from 5 to 21 days. In one embodiment the cultivating is for a total period of from 6 to 14 days.

In one embodiment the mitogenic stimulant is removed before the beginning of the second co-cultivation. In one embodiment the removal is by exchange of the cultivation supernatant and or washing the cells with neutral media.

One aspect as reported herein is a method for producing an antibody, which specifically binds to an antigen, comprising the following steps:
a) providing a population of antibody secreting (mature) rabbit B-cells,
b) staining the cells of the population of rabbit B-cells with at least one fluorescence dye (in one embodiment with one to three, or two to three fluorescence dyes),
c) depositing single cells of the stained population of rabbit B-cells or a pool of cells from the stained population of rabbit B-cells in individual containers (in one embodiment is the container a well of a multi well plate),
d) cultivating the deposited rabbit B-cells in the presence of rabbit CD40L expressing mammalian cells and IL-2 and IL-21,
e) determining the binding specificity of the antibodies secreted in the cultivation of the rabbit B-cells,
f) determining the amino acid sequence of the variable light and heavy chain domain of specifically binding antibodies by a reverse transcriptase PCR and nucleotide sequencing, and thereby obtaining a monoclonal antibody variable light and heavy chain domain encoding nucleic acid,
g) introducing the monoclonal antibody light and heavy chain variable domain encoding nucleic acid in an expression cassette for the expression of an antibody,
h) introducing the nucleic acid in a cell,
i) cultivating the cell and recovering the antibody from the cell or the cell culture supernatant and thereby producing an antibody, which specifically binds to an antigen.

In one embodiment the co-cultivating is in the presence of a mitogenic stimulant and/or an antibody production stimulant.

In one embodiment the cultivating is in the presence of IL-2 and IL-21 and IL-6.

In one embodiment the interleukins used in the co-cultivating are all human interleukins.

In one embodiment the pool of cells comprises of from about 10 B-cells to about 1,000,000 B-cells. In one embodiment the pool comprises of from about 500 B-cells to about 100,000 B-cells. In one embodiment the pool comprises about 500 B-cells.

One aspect as reported herein is a method for producing an antibody, which specifically binds to an antigen, comprising the following steps:
a) co-cultivating a pool of antibody secreting rabbit B-cells or a single deposited antibody secreting rabbit B-cell with rabbit CD40L expressing mammalian cells and IL-2 and IL-21,
b) isolating a nucleic acid encoding the amino acid sequence of the variable light chain domain and isolating a nucleic acid encoding the amino acid sequence of the variable heavy chain domain of an antibody, which specifically binds to an antigen,
c) cultivating a cell comprising the nucleic acid isolated in b) or a variant thereof encoding a humanized version of the light and/or heavy chain variable domain within one or more expression cassettes,
d) recovering the antibody from the cell or the cultivation medium and thereby producing an antibody, which specifically binds to an antigen.

In one embodiment the co-cultivating is in the presence of a mitogenic stimulant and/or an antibody production stimulant.

In one embodiment the method comprises the following steps:
a) providing a population of antibody secreting (mature) rabbit B-cells,
b) staining the cells of the population of B-cells with at least one fluorescence dye (in one embodiment with one to three, or two to three fluorescence dyes),
c) depositing single cells of the stained population of B-cells or a pool of cells from the stained population of B-cells in individual containers (in one embodiment is the container a well of a multi well plate),
d) cultivating the deposited individual B-cells in the presence of CD40L expressing mammalian cells and optionally IL-2 and IL-21,
e) determining the binding specificity of the antibodies secreted in the cultivation of the individual B-cells,
f) determining the amino acid sequence of the variable light and heavy chain domain of specifically binding antibodies by a reverse transcriptase PCR and nucleotide sequencing, and thereby obtaining a monoclonal antibody variable light and heavy chain domain encoding nucleic acid,
g) introducing the monoclonal antibody light and heavy chain variable domain encoding nucleic acid or a variant thereof encoding a humanized version of the light and/or heavy chain variable domain in an expression cassette for the expression of an antibody,
h) introducing the nucleic acid in a cell,
i) cultivating the cell and recovering the antibody from the cell or the cell culture supernatant and thereby producing an antibody, which specifically binds to an antigen.

In one embodiment the method comprises the following steps:
a) providing a population of antibody secreting (mature) rabbit B-cells,
b) staining the cells of the population of rabbit B-cells with at least one fluorescence dye (in one embodiment with one to three, or two to three fluorescence dyes),
c) depositing single cells of the stained population of rabbit B-cells or a pool of cells from the stained population of rabbit B-cells in individual containers (in one embodiment is the container a well of a multi well plate),
d) cultivating the deposited rabbit B-cells in the presence of rabbit CD40L expressing mammalian cells and IL-2 and IL-21,
e) determining the binding specificity of the antibodies secreted in the cultivation of the rabbit B-cells,
f) determining the amino acid sequence of the variable light and heavy chain domain of specifically binding antibodies by a reverse transcriptase PCR and nucleotide sequencing, and thereby obtaining a monoclonal antibody variable light and heavy chain domain encoding nucleic acid,
g) introducing the monoclonal antibody light and heavy chain variable domain encoding nucleic acid in an expression cassette for the expression of an antibody,
h) introducing the nucleic acid in a cell,
i) cultivating the cell and recovering the antibody from the cell or the cell culture supernatant and thereby producing an antibody, which specifically binds to an antigen.

In one embodiment the pool of cells comprises of from about 10 B-cells to about 1,000,000 B-cells. In one embodiment the pool comprises of from about 500 B-cells to about 100,000 B-cells. In one embodiment the pool comprises about 500 B-cells.

Reported herein is the use of a human CD40L expressing mammalian cell in the co-cultivation of antibody secreting human B-cells.

In one embodiment the mammalian cell is a BHK cell.

In one embodiment the human CD40L has the amino acid sequence of SEQ ID NO: 02.

Reported herein is the use of SAC in the co-cultivation of antibody secreting B-cells and CD40L expressing mammalian cells.

One aspect as reported herein is the use of a rabbit CD40L expressing BHK or CHO cell and IL-2 and IL-21 in a co-culture of a rabbit B-cell.

Reported herein is the use of a human CD40L expressing mammalian cell, and IL-2 and/or IL-21 and/or IL-6 in a co-culture of a human B-cell.

In one embodiment the co-culture is in the presence of IL-2 and IL-21.

In one embodiment the co-culture is in the presence of IL-2 and IL-21 and IL-6.

In one embodiment the interleukins used in the co-culture are all human interleukins.

One aspect as reported herein is a method for co-cultivating rabbit B-cells comprising the step of cultivating a single deposited rabbit B-cell or a pool of rabbit B-cells with rabbit CD40L expressing CHO or BHK cells in the presence of IL-2 and IL-21.

In one embodiment of all aspects as reported herein the IL-2 is either human IL-2 or mouse IL-2 and the IL-21 is either human IL-21 or mouse IL-21.

Reported herein is a method for co-cultivating human B-cells comprising the step of cultivating a single deposited human B-cell or a pool of human B-cells with human CD40L expressing mammalian cells in the presence of IL-2 and/or IL-21 and/or IL-6.

In one embodiment the co-cultivating is in the presence of IL-2 and IL-21.

In one embodiment the co-cultivating is in the presence of IL-2 and IL-21 and IL-6.

In one embodiment the interleukins used in the co-cultivating are all human interleukins.

One aspect as reported herein is the use of a rabbit CD40L expressing CHO or BHK cell, IL-2 and IL-21 in a co-culture of a rabbit B-cell for improving cell growth.

Reported herein is the use of a human CD40L expressing mammalian cell, IL-2 and/or IL-21 and/or IL-6 in a co-culture of a human B-cell for improving cell growth.

In one embodiment the co-culture is in the presence of IL-2 and IL-21.

In one embodiment the co-culture is in the presence of IL-2 and IL-21 and IL-6.

In one embodiment the interleukins used in the co-cultivating are all human interleukins.

Reported herein is a method for improving cell growth of rabbit B-cells comprising the step of cultivating a single deposited rabbit B-cell or a pool of rabbit B-cells with rabbit CD40L expressing mammalian cells in the presence of IL-2 and IL-21.

In one embodiment of all aspects as reported herein the IL-2 is either human IL-2 or mouse IL-2 and the IL-21 is either human IL-21 or mouse IL-21.

Reported herein is a method for improving cell growth of human B-cells comprising the step of cultivating a single deposited human B-cell or a pool of human B-cells with human CD40L expressing mammalian cells in the presence of IL-2 and/or IL-21 and/or IL-6.

In one embodiment the cultivating is in the presence of IL-2 and IL-21.

In one embodiment the cultivating is in the presence of IL-2 and IL-21 and IL-6.

In one embodiment the interleukins used in the co-cultivating are all human interleukins.

### Detailed Description of the Invention

The subject matter reported herein provides a generally applicable method for the rapid, efficient and reproducible generation of rabbit (or human) antibodies starting from rabbit (or human) B-cells and comprising at least one cultivation step.

It has been found that the addition of a rabbit CD40L expressing mammalian cell and IL-2 and IL-21 can improve the growth characteristics of rabbit B-cells, i.e. the rabbit B-cells, either a single deposited cell or as pool of cells, can be grown more rapidly to high cell densities than in the absence of the rabbit CD40L expressing mammalian cell and IL-2 and IL-21. Thus, it is possible to obtain high rabbit B-cell densities and correspondingly high IgG concentrations in the cultivation supernatant in short time.

Herein is reported as one aspect a CHO cell expressing rabbit CD40L.

One aspect as reported herein is the use of a rabbit CD40L expressing CHO cell together with IL-2 and IL-21 in the co-cultivation of rabbit B-cells.

Reported herein is a synthetic feeder mix that can be used in combination with the rabbit CD40L expressing CHO cell or other feeder cells to provide a highly efficient B-cell stimulation and cultivation system.

Herein is reported as one aspect a BHK cell expressing human CD40L.

Reported herein is the use of a human CD40L expressing BHK cell together with IL-2 and/or IL-21 in the co-cultivation of human B-cells.

Reported herein is a synthetic feeder mix that can be used in combination with the human CD40L expressing BHK cell or other feeder cells to provide a highly efficient B-cell stimulation and cultivation system.

Methods and techniques known to a person skilled in the art, which are useful for carrying out the current invention, are described e.g. in Ausubel, F.M., ed., Current Protocols in Molecular Biology, Volumes I to III (1997), Wiley and Sons; Sambrook, et al., Molecular Cloning: A Laboratory Manual, Second Edition, Cold Spring Harbor Laboratory Press, Cold Spring Harbor, New York (1989).

### Definitions

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The term "amino acid" as used within this application denotes the group of carboxy α-amino acids, which directly or in form of a precursor can be encoded by a nucleic acid. The individual amino acids are encoded by nucleic acids consisting of three nucleotides, so called codons or base-triplets. Each amino acid is encoded by at least one codon. The encoding of the same amino acid by different codons is known as "degeneration of the genetic code". The term "amino acid" as used within this application denotes the naturally occurring carboxy α-amino acids and is comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "clone" denotes a population of dividing and antibody secreting B-cells arising from/originating from a single B-cell. Thus, a B-cell clone produces a monoclonal antibody.

The term "expression" as used herein refers to transcription and/or translation processes occurring within a cell. The level of transcription of a nucleic acid sequence of interest in a cell can be determined on the basis of the amount of corresponding mRNA that is present in the cell. For example, mRNA transcribed from a sequence of interest can be quantitated by RT-PCR or by Northern hybridization (see Sambrook, et al., 1989, supra). Polypeptides encoded by a nucleic acid of interest can be quantitated by various methods, e.g. by ELISA, by assaying for the biological activity of the polypeptide, or by employing assays that are independent of such activity, such as Western blotting or radioimmunoassay, using immunoglobulins that recognize and bind to the polypeptide (see Sambrook, et al., 1989, supra).

An "expression cassette" refers to a construct that contains the necessary regulatory elements, such as promoter and polyadenylation site, for expression of at least the contained nucleic acid in a cell.

Expression of a gene is performed either as transient or as permanent expression. The polypeptide(s) of interest are in general secreted polypeptides and therefore contain an N-terminal extension (also known as the signal sequence) which is necessary for the transport/secretion of the polypeptide through the cell wall into the extracellular medium. In general, the signal sequence can be derived from any gene encoding a secreted polypeptide. If a heterologous signal sequence is used, it preferably is one that is recognized and processed (i.e. cleaved by a signal peptidase) by the host cell. For secretion in yeast for example the native signal sequence of a heterologous gene to be expressed may be substituted by a homologous yeast signal sequence derived from a secreted gene, such as the yeast invertase signal sequence, alpha-factor leader (including Saccharomyces, Kluyveromyces, Pichia, and Hansenula α-factor leaders, the second described in US 5,010,182), acid phosphatase signal sequence, or the C. albicans glucoamylase signal sequence (EP 0 362 179). In mammalian cell expression the native signal sequence of the protein of interest is satisfactory, although other mammalian signal sequences may be suitable, such as signal sequences from secreted polypeptides of the same or related species, e.g. for immunoglobulins from human or murine origin, as well as viral secretory signal sequences, for example, the herpes simplex glycoprotein D signal sequence. The DNA fragment encoding for such a pre-segment is ligated in frame, i.e. operably linked, to the DNA fragment encoding a polypeptide of interest.

The term "experimental animal" denotes a non-human animal. In one embodiment the experimental animal is selected from rat, mouse, hamster, rabbit, camel, llama, non-human primates, sheep, dog, cow, chicken, amphibians, sharks and reptiles. In one embodiment the experimental animal is a mammal.

The term "Fc-region" herein is used to define a C-terminal region of an immunoglobulin heavy chain that contains at least a portion of the constant region. The term includes native sequence Fc-regions and variant Fc-regions. In one embodiment, a human IgG heavy chain Fc-region extends from Cys226, or from Pro230, to the carboxyl-terminus of the heavy chain. However, the C-terminal lysine (Lys447) of the Fc-region may or may not be present. Unless otherwise specified herein, numbering of amino acid residues in the Fc-region or constant region is according to the EU numbering system, also called the EU index, as described in Kabat, E.A., et al., Sequences of Proteins of Immunological Interest, 5th ed., Public Health Service, National Institutes of Health, Bethesda, MD (1991), NIH Publication 91-3242, Vols. 1-3.

The term "feeder mix" denotes a combination of different additives, such as growth factors, cytokines and/or further proteins promoting the activation and/or survival of B-cells and/or antibody secretion. The feeder mix can be a natural feeder mix, e.g. obtained from the cultivation supernatant of thymocytes (TSN), which is a non-defined combination of cytokines, or the feeder mix can be a synthetic feeder mix, e.g. comprising a mixture of IL-21 and/or IL-2 and/or IL-6.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The term "cell" or "host cell" refers to a cell into which a nucleic acid, e.g. encoding a heterologous polypeptide, can be or is transfected. The term "cell" includes both prokaryotic cells, which are used for propagation of plasmids, and eukaryotic cells, which are used for the expression of a nucleic acid and production of the encoded polypeptide. In one embodiment, the eukaryotic cells are mammalian cells. In one embodiment the mammalian cell is a CHO cell, optionally a CHO K1 cell (ATCC CCL-61 or DSM ACC 110), or a CHO DG44 cell (also known as CHO-DHFR[-], DSM ACC 126), or a CHO XL99 cell, a CHO-T cell (see e.g. Morgan, D., et al., Biochemistry 26 (1987) 2959-2963), or a CHO-S cell, or a Super-CHO cell (Pak, S.C.O., et al. Cytotechnol. 22 (1996) 139-146). If these cells are not adapted to growth in serum-free medium or in suspension an adaptation prior to the use in the current method is to be performed. As used herein, the expression "cell" includes the subject cell and its progeny. Thus, the words "transformant" and "transformed cell" include the primary subject cell and cultures derived there from without regard for the number of transfers or sub-cultivations. It is also understood that all progeny may not be precisely identical in DNA content, due to deliberate or inadvertent mutations. Variant progeny that have the same function or biological activity as screened for in the originally transformed cell are included.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat, E.A., et al., Sequences of Proteins of Immunological Interest, 5th ed., Public Health Service, National Institutes of Health, Bethesda, MD (1991), NIH Publication 91-3242, Vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR", as used herein, refers to each of the regions of an antibody variable domain which are hypervariable in sequence and/or form structurally defined loops ("hypervariable loops"). Generally, native four-chain antibodies comprise six HVRs; three in the VH (HI, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia, C. and Lesk, A.M., J. Mol. Biol. 196 (1987) 901-917) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat, E.A., et al., Sequences of Proteins of Immunological Interest, 5th ed. Public Health Service, National Institutes of Health, Bethesda, MD (1991), NIH Publication 91-3242, Vols. 1-3.) With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro, J.C. and Fransson, J., Front. Biosci. 13 (2008) 1619-1633). Unless otherwise indicated, HVR residues and other residues in the variable domain (e.g., FR residues) are numbered herein according to Kabat et al., *supra.*

The term "labeling" denotes the presence or absence of a surface marker which can be determined by the addition of a specifically binding and labeled anti-surface marker antibody. Thus, the presence of a surface marker is determined e.g. in the case of a fluorescence label by the occurrence of a fluorescence whereas the absence of a surface marker is determined by the absence of a fluorescence after incubation with the respective specifically binding and labeled anti-surface marker antibody.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

A "nucleic acid" or a "nucleic acid sequence", which terms are used interchangeably within this application, refers to a polymeric molecule consisting of individual nucleotides (also called bases) a, c, g, and t (or u in RNA), for example to DNA, RNA, or modifications thereof. This polynucleotide molecule can be a naturally occurring polynucleotide molecule or a synthetic polynucleotide molecule or a combination of one or more naturally occurring polynucleotide molecules with one or more synthetic polynucleotide molecules. Also encompassed by this definition are naturally occurring polynucleotide molecules in which one or more nucleotides are changed (e.g. by mutagenesis), deleted, or added. A nucleic acid can either be isolated, or integrated in another nucleic acid, e.g. in an expression cassette, a plasmid, or the chromosome of a host cell. A nucleic acid is characterized by its nucleic acid sequence consisting of individual nucleotides.

To a person skilled in the art procedures and methods are well known to convert an amino acid sequence, e.g. of a polypeptide, into a corresponding nucleic acid sequence encoding this amino acid sequence. Therefore, a nucleic acid is characterized by its nucleic acid sequence consisting of individual nucleotides and likewise by the amino acid sequence of a polypeptide encoded thereby.

The term "specifically binding" and grammatical equivalents thereof denote that the antibody binds to its target with a dissociation constant (Kd) of 10⁻⁷ M or less, in one embodiment of from 10⁻⁸ M to 10⁻¹³ M, in a further embodiment of from 10⁻⁹ M to 10⁻¹³ M. The term is further used to indicate that the antibody does not specifically bind to other biomolecules present, i.e. it binds to other biomolecules with a dissociation constant (Kd) of 10⁻⁶ M or more, in one embodiment of from 10⁻⁶ M to 1 M.

A "transfection vector" is a nucleic acid (also denoted as nucleic acid molecule) providing all required elements for the expression of the in the transfection vector comprised coding nucleic acids/structural gene(s) in a host cell. A transfection vector comprises a prokaryotic plasmid propagation unit, e.g. for E. coli, in turn comprising a prokaryotic origin of replication, and a nucleic acid conferring resistance to a prokaryotic selection agent, further comprises the transfection vector one or more nucleic acid(s) conferring resistance to an eukaryotic selection agent, and one or more nucleic acid encoding a polypeptide of interest. Preferably are the nucleic acids conferring resistance to a selection agent and the nucleic acid(s) encoding a polypeptide of interest placed each within an expression cassette, whereby each expression cassette comprises a promoter, a coding nucleic acid, and a transcription terminator including a polyadenylation signal. Gene expression is usually placed under the control of a promoter, and such a structural gene is said to be "operably linked to" the promoter. Similarly, a regulatory element and a core promoter are operably linked if the regulatory element modulates the activity of the core promoter.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt, T.J., et al., Kuby Immunology, 6th ed., W.H. Freeman and Co., N.Y. (2007), page 91) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano, S., et al., J. Immunol. 150 (1993) 880-887; Clackson, T., et al., Nature 352 (1991) 624-628).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

The term "young animal" denotes an animal before sexual maturity occurs. A young hamster, for example, is of an age of less than 6 weeks, especially less than 4 weeks. A young mouse, for example, is of an age of less than 8 weeks, especially less than 5 weeks.

### Immunization

In the methods as reported herein B-cells obtained from e.g. mouse, rat, hamster, rabbit, or human can be used/processed.

The mouse can be an NMRI-mouse or a balb/c-mouse.

The hamster can be selected from Armenian hamster (Cricetulus migratorius), Chinese hamster (Cricetulus griseus), and Syrian hamster (Mesocricetulus auratus). The hamster can be the Armenia hamster.

In one embodiment the B-cell is a rabbit B-cell. In one embodiment the rabbit is selected from New Zealand White (NZW) rabbits, Zimmermann-rabbits (ZIKA), Alicia-mutant strain rabbits, basilea mutant strain rabbits, transgenic rabbits with a human immunoglobulin locus, rbIgM knock-out rabbits, and cross-breeding thereof. In one embodiment the rabbit B-cell is obtained from an immunized rabbit or from a rabbit after vaccination against a specific antigen.

The B-cell can be a human B-cell. The human B-cell can be obtained from a non-vaccinated, healthy human, or from a human after vaccination against a specific antigen, or from a human having a disease, or from a human that has survived a disease. The vaccination can be the administration of a specific vaccine to the human or the survival of a specific disease.

### Source and isolation of B-cells

The blood of an immunized experimental animal or of a vaccinated human provides a high diversity of antibody producing B-cells. The therefrom obtained B-cells secrete antibodies that have almost no identical or overlapping amino acid sequences within the CDRs and, thus, show a high diversity.

In one embodiment the B-cells of an experimental animal, e.g. obtained from the blood of the experimental animal, or from a human are obtained of from 4 days after immunization until 15 days after immunization or the most recent boost.

In one embodiment the B-cells are obtained after of from 4 days until at most 9 days after immunization or the most recent boost.

This time span allows for a high flexibility in the method as reported herein. In this time span it is likely that the B-cells providing for the most affine antibodies migrate from spleen to blood (see e.g. Paus, D., et al., JEM 203 (2006) 1081-1091; Smith, K.G.S., et al., The EMBO J. 16 (1997) 2996-3006; Wrammert, J., et al., Nature 453 (2008) 667-672).

### Selection steps prior to co-cultivation

B-cells producing antibodies that specifically bind an antigen can be enriched from peripheral blood mononuclear cells (PBMCs). Thus, in one embodiment of all methods as reported herein the B-cell is obtained from peripheral blood mononuclear cells (PBMCs) or the pool of B-cells is enriched from peripheral blood mononuclear cells (PBMCs).

Cells not producing an antibody binding the antigen of interest or, likewise, cells producing an antibody binding to the antigen of interest can be reduced or enriched, respectively, by using a panning approach. Therein a binding partner is presented attached to a surface and cells binding thereto are selectively enriched in the cell population in case the bound cells are processed further or reduced in the cell population in case the cells remaining in solution are processed further.

The method as reported herein comprises in one embodiment a selecting step in which B-cells producing specific antibodies are selected based on cell surface markers and fluorescence activated cell sorting/gating. In one embodiment mature B-cells are sorted/enriched/selected. For selection of B-cells from different experimental animal species different cell surface markers can be used.

With the labeling of non-target cell populations and non-specifically binding lymphocytes it is possible to selectively deplete these cells. In this depletion step generally a non-total depletion can be achieved. Albeit the depletion is not quantitative it provides for an advantage in the succeeding fluorescence labeling of the remaining cells as the number of interfering cells can be reduced or even minimized.

Different cell populations can be labeled by using different surface markers such as CD3⁺-cells (T-cells), CD19⁺-cells (B-cells in general), IgM⁺-cells (mature naive B-cells), IgG⁺-cells (mature B-cells), CD38⁺-cells (e.g. plasmablasts), and IgG⁺CD38⁺-cells (pre-plasma cells).

An immuno-fluorescence labeling for selection of mature IgG⁺-B-cells, such as memory B-cells, plasmablasts, and plasma cells can be used in the methods as reported herein.

For a selection or enrichment of B-cells the cells are either single labeled, or double labeled, or triple labeled.

It is required to perform a labeling that results in labeling about 0.1 % to 2.5 % of the total cell population.

In one embodiment B-cells are selected by the labeling of surface molecules present on 0.1 % to 2.5 % of the B-cells in the population. In one embodiment B-cells are selected by the labeling of surface molecules present on 0.3 % to 1.5 % of the B-cells in the population. In one embodiment B-cells are selected by the labeling of surface molecules present on 0.5 % to 1 % of the B-cells in the population.

### Single cell depositing

The methods as reported herein in one embodiment comprise the step of depositing the B-cells of a B-cell population as single cells.

In one embodiment the depositing as single cells is by fluorescence activated cell sorting (FACS).

In one embodiment specifically labeled B-cells are deposited as single cells. In one embodiment the labeling is a labeling of cell surface markers with fluorescence labeled antibodies.

In one embodiment the methods as reported herein provide for monoclonal antibodies.

In one embodiment of all methods as reported herein the B-cell is a mature B-cell and mature B-cells are deposited as single cells.

The immuno fluorescence labeling used for B-cells obtained from the blood of an experimental animal can also be used for the labeling of B-cells obtained from the spleen and other immunological organs of an experimental animal, such as mouse, rat, hamster, or rabbit.

### Multi cell depositing

The methods as reported herein in one embodiment comprise the step of depositing a pool of B-cells. In this pool of B-cells a total number of B-cells are deposited per well after magnetic affinity bead or FACS isolation from peripheral blood.

In one embodiment about 500 B-cells are deposited.

In one embodiment about 2,500 B-cells are deposited.

The total number of deposited B-cells can, e.g. for human B-cells, be increased up to 90,000 B-cells or more in a single experiment.

### Co-cultivation

Herein is reported the co-cultivation of antibody producing B-cells and CD40L expressing mammalian cells, such as CHO or BHK cells.

Thus, in one embodiment of all methods as reported herein the B-cells, either as pool of B-cells or as single deposited B-cells, are co-cultivated with the CD40L expressing CHO cells in the presence of IL-2 and IL-21 or a feeder mix.

Already after about 3, 4, 5, 6, 7, 8, 10, 15, or 21 days, especially after 3, 4 or 5 days (pool of about 2,500 B-cells), or especially after 6, 7 or 8 days (single deposited B-cell or pool of about 500 B-cells), of co-cultivation sufficient antibody molecules can be obtained in the supernatant. With the thereby provided amount of antibody different analyses can be performed in order to characterize the antibody, e.g. regarding binding specificity, in more detail. With the improved characterization of the antibody at this early stage in the screening / selection process it is possible to reduce the number of required nucleic acid isolations and sequencing reactions that have to be performed.

In one embodiment of all methods as reported herein is the co-cultivation of antibody producing B-cells and CD40L expressing CHO cells in the presence of a feeder mix.

The feeder mix can be a natural feeder mix or a synthetic feeder mix.

A natural feeder mix is e.g. thymocyte supernatant (TSN). It contains appropriate soluble factors, but this reagent is heterogeneous, the ingredients are not adequately described and differ from animal to animal.

A synthetic feeder mix is the so-called "Zubler feeder mix". It consists of a defined combination of murine cytokines (2 ng/ml IL-1β, 50 ng/ml IL-2, 10 ng/ml IL-10, and 2 ng/ml TNFα).

In one embodiment of all methods as reported herein the feeder mix is a thymocyte cultivation supernatant. In one embodiment the B-cell is not a human B-cell.

In one embodiment the thymocyte cultivation supernatant is obtained from the cultivation of thymocytes of the thymus gland of the respective young animal. It is especially suited to use the thymus gland of young animals compared to the isolation of thymocytes from the blood of adult animals.

In one embodiment the thymocyte cultivation supernatant is obtained from the cultivation of human T-cells obtained from the blood of a human.

In general the co-cultivation step of B-cells with the CD40L expressing CHO cells in the methods as reported herein can be preceded and also succeeded by a number of additional steps.

It has been found that the co-cultivation of rabbit CD40L expressing CHO cells and IL-2 and IL-21 and SAC can be used to activate early (non-mature or immature) rabbit B-cells. These B-cells do not express CD138.

In one embodiment of all methods as reported herein the co-cultivation of antibody producing B-cells and CD40L expressing CHO cells is in the presence of IL-2 and IL-21. In one embodiment the B-cell is a rabbit B-cell and the CD40L is rabbit CD40L. In one embodiment the cultivation medium is essentially free of IL-4, i.e. IL-4 is not added to the cultivation medium. In one embodiment IL-2 and IL-21 are added at the start of the cultivation together with the cultivation medium.

In one embodiment of all methods as reported herein the co-cultivation of antibody producing rabbit B-cells and rabbit CD40L expressing CHO cells is in the presence of IL-2 and IL-21, whereby the cultivation medium is essentially free of IL-4, and whereby IL-2 and IL-21 are added at the start of the cultivation together with the cultivation medium.

It has been found that the use of a co-cultivation system comprising a B-cell, a CD40L expressing mammalian cell, IL-2 and IL-21 results in an improved proliferation of the B-cell, i.e. the B-cell divides more rapidly and higher cell densities and antibody titer in the cultivation supernatant, respectively, can be obtained in shorter time compared to a system not comprising one, two, or all of IL-2, IL-21, and a CD40L expressing mammalian cell.

It has further been found that for rabbit B-cells a co-cultivation system comprising rabbit CD40L expressing CHO cells, human or murine IL-2 and human or murine IL-21 is especially suited.

### Characterization of co-cultivated cells by IgG production

For the (qualitative and quantitative) determination of secreted IgG after the co-cultivation generally all methods known to a person of skill in the art such as an ELISA can be used. In one embodiment of all methods as reported herein an ELISA is used. For determination of human IgG levels the Cytometric Bead Array (CBA) - technology (available from BD Biosciences) has been used.

Depending on the characterization results a B-cell clone can be obtained, i.e. selected.

### Characterization of co-cultivated cells by expansion and proliferation

For the (qualitative and quantitative) determination of cellular expansion the proliferation or viability of the co-cultivated B-cells can be assessed using different readout systems (commercial cellular activity test "cell titer glow" (Promega), the CFSE dilution method, ^{3H}Thymidin incorporation, or cell culture images).

### Isolation of mRNA, cloning and sequencing

A B-cell clone producing high antibody titers provides an amount of mRNA encoding (cognate) monoclonal light and heavy chain variable region allowing the use of degenerated PCR primer and obviates the requirement of highly specific PCR primer. Also the required number of PCR cycles is reduced. Thus, in one embodiment the reverse transcriptase PCR is with degenerated PCR primer for the light and heavy chain variable domain.

From the B-cells the total mRNA can be isolated and transcribed in cDNA. With the respective primer the cognate VH- and VL-region encoding nucleic acid can be amplified.

In one embodiment of all methods as reported herein the amino acid sequence is derived from the amplified variable domain-encoding nucleic acid. The exact start and end point of the variable domain-encoding nucleic acid is identified by locating the amino acid sequences of EVQL/QVQL to VSS (VH-region) and DIVM/DIQM to KLEIK (VL-region).

### Reported herein

Herein reported is the co-stimulation of antibody secreting B-cells (i) via the CD40/CD40L (CD154) pathway using irradiated mammalian, e.g. CHO (Chinese hamster ovary) or BHK (baby hamster kidney), feeder cells transfected with CD40L, e.g. of rabbit origin or of human origin, and (ii) via supplementation of the cultivation medium with individual cytokines or a feeder mix comprising e.g. human or mouse Interleukin 2 (IL-2) and 21 (IL-21), and/or heat-killed, formalin-fixed Staphylococcus A strain Cowan 1 particles (SAC).

### Aspects and embodiments of the current invention

One aspect as reported herein is a method for producing an antibody comprising the step of co-cultivating a rabbit B-cell with a rabbit CD40L expressing CHO or BHK cell in the presence of IL-2 and IL-21.

With the co-cultivation step of rabbit B-cells (single deposited cell or a pool of cells) with a rabbit CD40L expressing, e.g. cell surface presenting, mammalian cell in the presence of IL-2 and IL-21 a rapid, efficient and reproducible method for the generation of rabbit or human antibodies starting from rabbit B-cells is provided.

In one embodiment the rabbit B-cell is a naive rabbit B-cell or a non-mature rabbit B-cell.

In one embodiment the rabbit B-cell is an IgG⁺ B-cell.

In one embodiment the IL-2 is human IL-2. In one embodiment the IL-2 is murine IL-2.

In one embodiment the IL-21 is human IL-21. In one embodiment the IL-21 is murine IL-21.

In one embodiment the IL-2 is human IL-2 and the IL-21 is murine IL-21.

In one embodiment the IL-2 is murine IL-2 and the IL-21 is murine IL-21.

In one embodiment the IL-2 is human IL-2 and the IL-21 is human IL-21.

In one embodiment the IL-2 is murine IL-2 and the IL-21 is human IL-21.

In one embodiment the cultivation is in the absence of IL-4. In one embodiment the cultivation medium is free of IL-4.

In one embodiment the mammalian cell is a Chinese hamster ovary cell.

In one embodiment the rabbit CD40L has the amino acid sequence of SEQ ID NO: 01.

In one embodiment the rabbit B-cells are obtained after 4 to 9 days after the last immunization or boost of the rabbit with the antigen.

In one embodiment the method further comprises one or more of the following steps:
- obtaining B-cells from an immunized rabbit, and/or
- single depositing the B-cells, and/or
- co-cultivating the B-cells with a rabbit CD40L expressing CHO or BHK cell, and/or
- obtaining the nucleic acid encoding the variable domains of the rabbit antibody from the co-cultured rabbit B-cells, and/or
- humanizing the variable domains of the rabbit antibody, and/or
- cultivating a mammalian cell comprising a nucleic acid encoding the variable domains of the antibody and recovering the antibody from the cell or the cultivation medium.

Reported herein is a method for producing an antibody comprising the step of co-cultivating a human B-cell with a human CD40L expressing mammalian cell.

With the co-cultivation step of human B-cells (single deposited cell or pool of cells) with a human CD40L expressing, e.g. cell surface presenting, mammalian cell an efficient and reproducible method for the generation of human antibodies starting from human B-cells is provided.

In one embodiment the human B-cell is obtained from the blood of a human.

In one embodiment the rabbit B-cell is an IgG⁺ B-cell.

In one embodiment the mammalian cell is a baby hamster kidney cell.

In one embodiment IL-2 and/or IL-21 and/or IL-6 is/are added to the co-cultivation.

In one embodiment the IL-2 is human IL-2.

In one embodiment the IL-21 is human IL-21.

In one embodiment the IL-6 is human IL-6.

In one embodiment the cultivation is in the absence of IL-4. In one embodiment the cultivation medium is free of IL-4.

In one embodiment the human CD40L has the amino acid sequence of SEQ ID NO: 02.

In one embodiment the B-cells are obtained after 4 to 9 days after the immunization the human with the antigen or a vaccine.

In one embodiment the method further comprises one or more of the following steps:
- obtaining B-cells from the blood of an immunized, or vaccinated, or disease having, or disease surviving human, and/or
- single depositing the B-cells, and/or
- co-cultivating the B-cells with a human CD40L expressing mammalian cell, and/or
- obtaining the nucleic acid encoding the variable domains of the human antibody from the co-cultured human B-cells, and/or
- cultivating a mammalian cell comprising a nucleic acid encoding the variable domains of the human antibody and optionally a nucleic acid encoding the constant region of a human antibody and recovering the antibody from the cell or the cultivation medium.

Reported herein is the use of IL-2 and IL-21 for the co-cultivation of B-cells with feeder cells.

The addition of IL-2 and IL-21 to the co-cultivation of a B-cell with a feeder cell provides for a highly efficient B-cell stimulation and improved growth characteristics, such as improved growth rate and/or antibody secretion.

In one embodiment the feeder cell is a CD40L expressing mammalian cell.

In one embodiment the B-cell is a rabbit B-cell and the feeder cell is a rabbit CD40L expressing mammalian cell. In one embodiment the mammalian cell is a Chinese hamster ovary cell.

Reported herein is the use of IL-2 and/or IL-21 for the co-cultivation of B-cells with feeder cells.

The addition of IL-2 and/or IL-21 and/or IL-6 to the co-cultivation of a B-cell with a feeder cell provides for a highly efficient B-cell stimulation and improved growth characteristics, such as improved growth rate and/or antibody secretion.

In one embodiment the feeder cell is a CD40L expressing mammalian cell.

In one embodiment the B-cell is a human B-cell and the feeder cell is a human CD40L expressing mammalian cell. In one embodiment the mammalian cell is a baby hamster kidney cell.

One aspect as reported herein is a method for producing an antibody comprising the step of co-cultivating a B-cell with feeder cells and IL-2 and IL-21.

In one embodiment the feeder cell is a CD40L expressing mammalian cell.

In one embodiment the B-cell is a rabbit B-cell and the feeder cell is a rabbit CD40L expressing mammalian cell. In one embodiment the mammalian cell is a Chinese hamster ovary cell.

Reported herein is a method for producing an antibody comprising the step of co-cultivating a B-cell with feeder cells and IL-2 and/or IL-21 and/or IL-6.

In one embodiment the feeder cell is a CD40L expressing mammalian cell.

In one embodiment the B-cell is a human B-cell and the feeder cell is a human CD40L expressing mammalian cell. In one embodiment the mammalian cell is a baby hamster kidney cell.

Reported herein is a kit comprising rabbit CD40L expressing CHO cells, IL-2 and IL-21.

Reported herein is a kit comprising human CD40L expressing BHK cells and IL-2 or IL-21 or IL-6 or a combination thereof.

Reported herein is a method for cultivating a B-cell secreting an antibody that specifically binds to a T-cell surface antigen and that mediates a negative stimulus to T-cells comprising the co-cultivation of the B-cell and a CD40L expressing mammalian cell in the presence of IL-2 or IL-21 or both.

This co-cultivation system is useful if a B-cell, which is secreting a T-cell-inhibiting compound, has to be amplified starting from a single or pooled B-cell and feeder cells derived from T-cells, e.g. thymoma cells, cannot be used as the secreted T-cell-inhibiting compound would reduce or abolish the effect of the feeder cell.

The CD40L expressing CHO cells as reported herein can be used for the generation of T-cell-specific antibodies. The use of a feeder system consisting of T-cells or comprising T-cell antigens, such as the thymoma cell line EL4-B5, are not suited as the T-cell-specific antibodies produced by the B-cells would interfere with the culture system. For the cultivation of T-cell-specific antibody secreting (B-)cells an artificial, T-cell independent system would be ideal, such as a system as reported herein.

In one embodiment the antigen is not CD40L.

In one embodiment the T-cell-inhibiting compound is an antibody that specifically binds to a T-cell surface antigen.

In one embodiment the B-cell is a rabbit B-cell and the feeder cell is a rabbit CD40L expressing mammalian cell. In one embodiment the mammalian cell is a Chinese hamster ovary cell.

In one embodiment IL-2 and IL-21 are added to the co-cultivation.

In one embodiment the B-cell is a human B-cell and the feeder cell is a human CD40L expressing mammalian cell. In one embodiment the mammalian cell is a baby hamster kidney cell.

In one embodiment IL-2 and/or IL-21 and/or IL-6 is/are added to the co-cultivation.

Reported herein is a method for the cultivation of an antibody secreting B-cell comprising in the following order
i) a first co-cultivation of the B-cell and a CD40L expressing mammalian cell in the presence of a mitogenic stimulant, and
ii) a subsequent second co-cultivation of the B-cell and the CD40L expressing mammalian cell in the presence of an antibody production stimulant.

With the sequential cultivation of a B-cell first in the presence of a mitogenic stimulant and thereafter in the presence of an antibody production stimulant the co-cultivation of the B-cell is separated in a first phase in which the B-cell is amplified and in a second phase in which the antibody secretion is induced.

In one embodiment the first and second co-cultivation are performed in the same cultivation vessel.

In one embodiment the method for the cultivation of antibody secreting B-cells comprises a co-cultivation of the B-cell and a CD40L expressing mammalian cell whereby at first a mitogenic stimulant is added to the cultivation and thereafter an antibody production stimulant is added to the cultivation.

In one embodiment the antibody production stimulant is added at least one hour after the mitogenic stimulant is added. In one embodiment the antibody production stimulant is added one to three days after the mitogenic stimulant is added to the cultivation.

In one embodiment the mitogenic stimulant is selected from the group comprising CD40- and CD40L-interacting compounds, ICOS- and ICOS-L-interacting compounds, APRIL, BAFF, CR2, CXCL9, CXCL12 (SDF-1), CXCL13, CXCL16, Flt-3L, Interleukin-1 (α/β), Interleukin-2, Interleukin-3, Interleukin-4, Interleukin-5, Interleukin-7, Interleukin-10, Interleukin-14, Interleukin-21, SAP (signaling lymphocyte activation molecule [SLAM] associated protein), Staphylococcus A strain Cowan 1 particles (SAC; heat-killed, formalin-fixed), TLR Ligands such as LPS, different CpG ODNs or Resiquimod (R-848), TSLP, Tumor necrosis factor (TNF) alpha, type I Interferons (e.g. IFN α/β), type II interferon (e.g. IFNγ), cross-linking anti-IgG antibody, cross-linking anti-CD20 antibody, and cross-linking anti-CD27 antibody.

In one embodiment the antibody production stimulant is selected from the group comprising CD40- and CD40L-interacting compounds, ICOS- and ICOS-L-interacting compounds, APRIL, BAFF, CR2, CXCL9, CXCL12 (SDF-1), CXCL13, CXCL16, Flt-3L, Interleukin-1 (α/β), Interleukin-2, Interleukin-3, Interleukin-4, Interleukin-5, Interleukin-6, Interleukin-9, Interleukin-10, Interleukin-13, Interleukin-21, Interleukin-33, SAP (signaling lymphocyte activation molecule [SLAM] associated protein), Staphylococcus A strain Cowan 1 particles (SAC; heat-killed, formalin-fixed), TLR Ligands such as LPS, different CpG ODNs or Resiquimod (R-848), TSLP, Tumor necrosis factor (TNF) alpha, type I Interferons (e.g. IFN α/β), type II interferon (e.g. IFNγ), cross-linking anti-IgG antibody, cross-linking anti-CD20 antibody, and cross-linking anti-CD27 antibody. In one embodiment the mitogenic stimulant is added at the beginning of the first co-cultivation.

In one embodiment the mitogenic stimulant is added 1, 2, 3, 4, 5, 6, 7, 8, 9, 10, 11, 12, 13, or 14 days after the beginning of the first co-cultivation.

In one embodiment the antibody production stimulant is added 1, 2, 3, 4, 5, 6, 7, 8, 9, 10, 11, 12, 13, or 14 days after the addition of the mitogenic stimulant.

In one embodiment each of the co-cultivations is performed for 5 to 14 days.

In one embodiment the cultivating is for a total period of from 5 to 21 days. In one embodiment the cultivating is for a total period of from 6 to 14 days.

In one embodiment the mitogenic stimulant is removed before the beginning of the second co-cultivation. In one embodiment the removal is by exchange of the cultivation supernatant and or washing the cells with neutral media.

One aspect as reported herein is a method for producing an antibody, which specifically binds to an antigen, comprising the following steps:
a) co-cultivating a pool of or single deposited antibody secreting B-cell with CD40L expressing mammalian cells,
b) cultivating a cell comprising a nucleic acid encoding the variable regions or a variant thereof of the antibody secreted by the B-cell co-cultivated in step a) within one or more expression cassettes,
c) recovering the antibody from the cell or the cultivation medium and thereby producing an antibody, which specifically binds to an antigen.

In one embodiment the co-cultivating is in the presence of a mitogenic stimulant and/or an antibody production stimulant.

In one embodiment the first and/or second co-cultivating is in the presence of IL-2 and IL-21.

In one embodiment the IL-2 is human IL-2. In one embodiment the IL-2 is murine IL-2.

In one embodiment the IL-21 is human IL-21. In one embodiment the IL-21 is murine IL-21.

In one embodiment the IL-2 is human IL-2 and the IL-21 is murine IL-21.

In one embodiment the IL-2 is murine IL-2 and the IL-21 is murine IL-21.

In one embodiment the IL-2 is human IL-2 and the IL-21 is human IL-21.

In one embodiment the IL-2 is murine IL-2 and the IL-21 is human IL-21.

In one embodiment the cultivation is in the absence of IL-4. In one embodiment the cultivation medium is free of IL-4.

In one embodiment comprises the co-cultivating
i) a first co-cultivation of the B-cell and a CD40L expressing mammalian cell in the presence of a mitogenic stimulant, and
ii) a subsequent second co-cultivation of the B-cell and the CD40L expressing mammalian cell in the presence of an antibody production stimulant.

In one embodiment comprises the method the following step
ab) isolating a nucleic acid encoding the amino acid sequence of the variable light chain domain and isolating a nucleic acid encoding the amino acid sequence of the variable heavy chain domain of an antibody, which specifically binds to an antigen.

In one embodiment the method comprises the following steps:
a) providing a population of antibody secreting (mature) B-cells,
b) staining the cells of the population of B-cells with at least one fluorescence dye,
c) depositing single cells of the stained population of B-cells in individual containers,
d) cultivating the deposited individual B-cells in the presence of CD40L expressing mammalian cells and IL-2 and IL21,
e) determining the binding specificity of the antibodies secreted in the cultivation of the individual B-cells,
f) determining the amino acid sequence of the variable light and heavy chain domain of specifically binding antibodies by a reverse transcriptase PCR and nucleotide sequencing, and thereby obtaining a monoclonal antibody variable light and heavy chain domain encoding nucleic acid,
g) introducing the monoclonal antibody light and heavy chain variable domain encoding nucleic acid in an expression cassette for the expression of an antibody,
h) introducing the nucleic acid in a cell,
i) cultivating the cell and recovering the antibody from the cell or the cell culture supernatant and thereby producing an antibody, which specifically binds to an antigen.

In one embodiment the B-cells are obtained from the blood of an animal or a human.

In one embodiment the staining is with one to three, or two to three fluorescence dyes.

In one embodiment the container is a well of a multi well plate.

In one embodiment the cell is a mammalian cell. In one embodiment the mammalian cell is a CHO cell, or a BHK cell, or a NS0 cell, or a Sp2/0 cell.

One aspect as reported herein is the use of a rabbit CD40L expressing CHO or BHK cell, IL-2 and IL-21 in the co-cultivation of antibody secreting rabbit B-cells.

In one embodiment the mammalian cell is a CHO cell.

In one embodiment the IL-2 is human IL-2. In one embodiment the IL-2 is murine IL-2.

In one embodiment the IL-21 is human IL-21. In one embodiment the IL-21 is murine IL-21.

In one embodiment the IL-2 is human IL-2 and the IL-21 is murine IL-21.

In one embodiment the IL-2 is murine IL-2 and the IL-21 is murine IL-21.

In one embodiment the IL-2 is human IL-2 and the IL-21 is human IL-21.

In one embodiment the IL-2 is murine IL-2 and the IL-21 is human IL-21.

In one embodiment the cultivation is in the absence of IL-4. In one embodiment the cultivation medium is free of IL-4.

In one embodiment the rabbit CD40L has the amino acid sequence of SEQ ID NO: 01.

Reported herein is the use of a human CD40L expressing mammalian cell together with IL-2 and/or IL-21 and/or IL-6 in the co-cultivation of antibody secreting human B-cells.

In one embodiment the mammalian cell is a BHK cell.

In one embodiment the human CD40L has the amino acid sequence of SEQ ID NO: 02.

Reported herein is the use of SAC in the co-cultivation of antibody secreting B-cells.

In one embodiment the co-cultivation is with CD40L expressing mammalian cells and/or IL-2.

The following examples, figures and sequences are provided to aid the understanding of the present invention, the true scope of which is set forth in the appended claims. It is understood that modifications can be made in the procedures set forth without departing from the spirit of the invention.

### Description of the Figures

- **Figure 1**: Proliferation of B-cells after co-cultivation of 2,500 purified rabbit B-cells with 5,000 γ-irradiated rabbit CD40L expressing CHO cells or EL4-B5 cells for 7 days.
- **Figure 2**: Microscopic images of co-cultivation of B-cells and feeder cells in the absence or presence of different stimuli after 6 days of co-cultivation: a) EL4-B5 cells and Zubler mix, b) rbCD40L expressing CHO cells and human IL-21, c) rbCD40L expressing CHO cells and human IL-2, d) wt-CHO cells and human IL-21 and human IL-2, e) rbCD40L expressing CHO cells and human IL-21 and human IL-2, f) rbCD40L expressing CHO cells and human IL-21 and human IL-2 and SAC.
- **Figure 3**: Seven hundred fifty sorted human IgG⁺ memory B-cells per well were co-cultured with 5,000 γ-irradiated CD40L expressing BHK cells and indicated feeder mixes. Shown is the proliferation of isolated human memory B-cells after 8 days.
- **Figure 4**: Proliferation of untouched isolated human B-cells. After isolation from PBMC, 90,000 pooled human B-cells per well were co-cultured with γ-irradiated CD40L expressing BHK cells and indicated feeder mixes. Shown is the proliferation (absolute cell numbers) after 7 days.
- **Figure 5**: Dotplots showing CFSE-dilution as a degree of the proliferation analyzed by FACS. Shown is the CFSE staining (X-axis) of gated CD19⁺ B-cells (Y-axis).
- **Figure 6**: B-cell clusters (cultivated without feeder cells) after 3 days of incubation. Microscopic images of human B-cells cultured three days alone (a) or in the presence of human IL-2 (b) or IL-2 and SAC (c).
- **Figure 7**: Five hundred sorted rabbit IgG⁺ B-cells per well were cocultured with 10,000 γ-irradiated rabbit CD40L expressing CHO cells and IL-2 and IL-21 of different origin and combinations thereof. Shown is the antibody titer after 7 days in the culture supernatant (median); 1: mu IL-2, 2: hu IL-2, 3: mu IL-21, 4: hu IL-21, 5: hu IL-2 and hu IL-21, 6: hu IL-2 and mu IL-21, 7: mu IL-2 and hu IL-21, 8: mu IL-2 and mu IL-21; γ-axis: rb IgG [µg/ml].
- **Figure 8**: Result of the CTG viability assay for the co-cultivation of Figure 7.
- **Figure 9**: Five hundred thawed sorted rabbit IgG⁺ B-cells per well were co-cultured with 10,000 γ-irradiated CHO-K1 cells (below detection limit) or with 10,000 γ-irradiated rabbit CD40L expressing CHO cells and IL-2 and IL-21 of different origin and combinations thereof. Shown is the antibody titer after 7 days in the culture supernatant (median); 1: mu IL-2, 2: hu IL-2, 3: mu IL-21, 4: hu IL-21, 5: hu IL-2 and hu IL-21, 6: hu IL-2 and mu IL-21, 7: mu IL-2 and hu IL-21, 8: mu IL-2 and mu IL-21; γ-axis: rb IgG [µg/ml].
- **Figure 10**: Result of the CTG viability assay for the co-cultivation of Figure 9.
- **Figure 11**: Five hundred sorted rabbit IgG⁺ B-cells per well were cocultured with 10,000 γ-irradiated CHO-K1 cells (right bars; not visible as below detection limit) or with 10,000 γ-irradiated rabbit CD40L expressing CHO cells (left bars) and IL-2 and IL-21 of different origin and combinations thereof. Shown is the antibody titer after 7 days in the culture supernatant (mean).
- **Figure 12**: Result of the CTG viability assay for the co-cultivation of Figure 11.

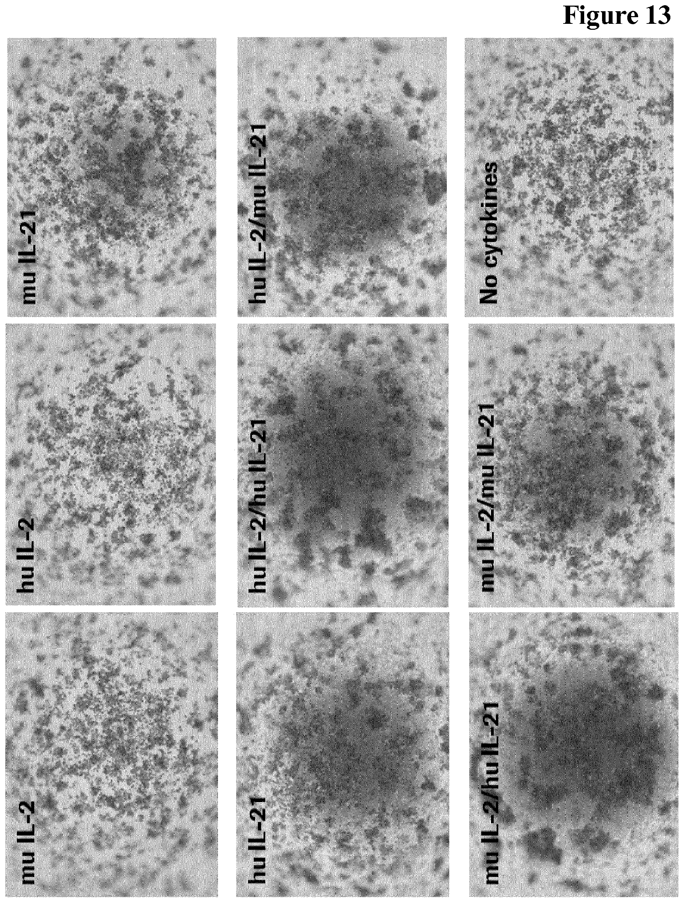

- **Figure 13**: Microscopic images of co-cultivation of B-cells and feeder cells of Example 14 at low B-cell density (500 B-cells).
- **Figure 14**: Two thousand five hundred sorted rabbit IgG⁺ B-cells per well were co-cultured with 10,000 γ-irradiated CHO-K1 cells (right bars) or 10,000 γ-irradiated rabbit CD40L expressing CHO cells (left bars) and IL-2 and IL-21 of different origin and combinations thereof. Shown is the antibody titer after 7 days in the culture supernatant (mean).

### Description of the sequences

**SEQ ID NO: 01** amino acid sequence of rabbit CD40L
**SEQ ID NO: 02** amino acid sequence of human CD40L
**SEQ ID NO: 03** amino acid sequence of mouse CD40L

### Examples

### Material & Methods

### Recombinant DNA techniques

Standard methods were used to manipulate DNA as described in Sambrook, J., et al., Molecular Cloning: A Laboratory Manual, Cold Spring Harbor Laboratory Press, Cold Spring Harbor, New York (1989). The molecular biological reagents were used according to the manufacturer's instructions.

### DNA sequence determination

DNA sequences were determined by double strand sequencing performed at SequiServe GmbH (Vaterstetten, Germany).

### DNA and protein sequence analysis and sequence data management

The GCG's (Genetics Computer Group, Madison, Wisconsin) software package variant 10.2 and Infomax's Vector NTI Advance suite variant 8.0 was used for sequence creation, mapping, analysis, annotation and illustration.

### Gene synthesis

Desired gene segments encoding cDNA of rabbit CD40L were prepared by Geneart GmbH (Regensburg, Germany). The gene segments are flanked by singular restriction endonuclease cleavage sites to facilitate expression construct cloning as described below. The DNA sequence of the subcloned gene fragments were confirmed by DNA sequencing.

### Cytokines

| | |
|---|---|
| Zubler Mix: | 2 ng/ml mouse IL-1β, 50 ng/ml mouse IL-2, 10 ng/ml mouse IL-10, and 2 ng/ml mouse TNFα (final concentration) |

Cytokines tested for establishment of a defined cytokine cocktail (given as final concentration in case not stated otherwise):

| cytokine | final concentration | supplier | Catnr. |
|---|---|---|---|
| huIL-2 | 50 U/ml | Roche Dia. GmbH | 11147528001 |
| huIL-21 | 25 ng/ml | eBioscience | 14-8219 |
| muIL-21 | 100 ng/ml | R&Dsystems | 594-ML |
| huIL-6 | 300 U/ml | Roche Dia. GmbH | 11138600001 |
| huIL-10 | 25 ng/ml | BD | 554611 |
| huIL-1β | 12.5 ng/ml | R&Dsystems | 201-LB |
| huIL-33 | 100 ng/ml | Peprotech | 200-33 |
| TNFα | 25 ng/ml | R&Dsystems | 210-TA |

### CHO rbCD40L medium

| | | |
|---|---|---|
| 500 ml HyQSFM4CHO | # SH30549 | Perbio |
| 50 ml FCS | # A15-512 | PAA |
| 1 ml Pen/Strep | # 11074440001 | Roche Dia. GmbH |
| 5 ml Hygromycin | # 10843555-001 | Roche Dia. GmbH |

### Rabbit B-cell medium

| | | |
|---|---|---|
| 500 ml RPMI 1640 | #P04-17500 | PAN Biotech |
| 50 ml FCS | #A15-512 | PAA |
| 5 ml L-Gln | #25030-024 | Invitrogen |
| 5 ml potassium Pyruvate | #P04-43100 | PAN Biotech |
| 5 ml HEPES | #15630-056 | Invitrogen |
| 500 µl β-Mercaptoethanol | # 31350010 | Invitrogen |
| 1 ml Pen/Strep | #11074440001 | Roche Dia. GmbH |

### Additives to rabbit B-cell medium

| | | |
|---|---|---|
| SAC | #507858 | Calbiochem |
| IL-21 | #14-8219 | eBioscience |
| IL-2 | #1114752800 | Roche |
| | | |
| 96er U-plate | #3799 | Corning |

### Phenotyping/sorting of antibodies

| | | |
|---|---|---|
| goat anti-rabbit IgG Fc-antibody | AbDSerotec | STAR121F |
| rat anti-rabbit CD138-antibody | Roche GlycArt AG | |
| anti-human CD40 mAb (clone Mab89) | Beckman Coulter | IM1374 |
| anti human/murine (rabbit cross-reactive) | | |
| anti CD40L antibody: | | |
| anti-muCD40L antibody | R&D systems | AF1163 |
| anti-huCD40L antibody | &D systems | AF617 R |
| donkey anti-goat IgG antibody Alexa 488 | Molecular Probes | A11055 |

### Miscellaneous

| | | |
|---|---|---|
| anti-FITC antibody-coupled microbeads Miltenyi Biotec | | #130-048-701 |
| human B-cell negative isolation kit | Invitrogen | #113.13D |
| Nucleofector Kit T | Lonza | VCA-1002 |
| CBA for total IgG | BD Biosciences | #558679 |

### Animals

Wild-type New Zealand White Rabbits were used as a source of blood. They were housed and maintained according to the Institutional Animal Care and Use committee guidelines and Association for Assessment and Accreditation of Laboratory Animal Care (Germany, Europe).

### Coating of plates

Sterile streptavidin-coated 6-well plates (cell culture grade) were incubated with biotinylated antigens in a concentration at 0.5-1 µg/ml in PBS at room temperature for one hour. Sterile cell culture 6-well plates were coated with 2 µg/ml non-biotinylated protein antigen in carbonate buffer (0.1 M sodium bicarbonate, 34 mM Disodiumhydrogencarbonate, pH 9.55) over night at 4 °C.
Plates were washed in sterile PBS three times before use.

### Isolation of rabbit peripheral blood mononuclear cells (PBMC)

EDTA containing whole blood was diluted twofold with 1xPBS before density centrifugation on lympholyte mammal (Cedarlane Laboratories) or Ficoll Paque Plus (GE Healthcare, cat. #17-1440-03), which was performed to isolate rabbit PBMC. PBMCs were washed twice before staining with antibodies.

### EL4-B5 medium

RPMI 1640 (Pan Biotech, Aidenbach, Germany) supplemented with 10 % FCS (Hyclone, Logan, UT, USA), 2 mM Glutamine, 1 % penicillin/streptomycin solution (PAA, Pasching, Austria), 2 mM sodium pyruvate, 10 mM HEPES (PAN Biotech, Aidenbach, Germany) and 0.05 mM β-mercaptoethanol (Gibco, Paisley, Scotland)

### Depletion of macrophages/monocytes

Sterile 6-well plates (cell culture grade) were used to deplete macrophages and monocytes through unspecific adhesion. Each well was filled at maximum with 4 ml media and up to 6x10⁶ peripheral blood mononuclear cells from the immunized rabbit and allowed to bind for one hour at 37 °C in the incubator. 50 % of the cells in the supernatant were used for the panning step; the remaining 50 % of cells were kept on ice until the immune fluorescence staining.

### Enrichment of B-cells on the protein antigen

Six-well tissue culture plates coated with the protein antigen were seeded with up to 6x10⁶ cells per 4 ml medium and allowed to bind for one hour at 37 °C in the incubator. After the enrichment step on the protein antigen non-adherent cells were removed by carefully washing the wells 1-2 times with 1xPBS. The remaining sticky cells were detached by trypsin for 10 min at 37 °C in the incubator and then washed twice in media. The cells were kept on ice until the immune fluorescence staining.

### Immune fluorescent staining and Flow cytometry (sorting and analysis)

Anti-rabbit IgG FITC used for single cell sorting was from AbD Serotec (STAR121F, Düsseldorf, Germany).

For surface staining, cells were incubated with the optimally diluted anti-rabbit IgG FITC antibody in PBS for 30 min. with rolling at 4 °C in the dark. Following centrifugation, the supernatants were removed by aspiration. The PBMCs were subjected to two cycles of centrifugation and washing with ice cold PBS. Finally the PBMCs were resuspended in ice cold PBS and immediately subjected to the FACS analyses. Propidium iodide in a concentration of 5 µg/ml (BD Pharmingen, San Diego, CA, USA) was added prior to the FACS analyses to discriminate between dead and live cells. In other experiments the stained cells were single deposited by FACS.

A Becton Dickinson FACSAria equipped with a computer and the FACSDiva software (BD Biosciences, USA) were used to collect and analyze the data.

### Proliferation assays

### a) Cell Titer Glo (CTG) viability assay

The CTG viability assay (Promega; #G7571) was used according to the instructions of the manufacturer.

### b) ³H Thymidine Assay

After 6 days of incubation ³H-Thymidin was added (0.5 µCi/well) and incubated for further 16 hours. The incorporation of ³H-Thymidine during cell proliferation was determined with a microplate scintillation counter (Wallac).

### c) Microscopic analysis

For the acquisition of microscopic images, a phase contrast microscope from Leica (Leica DM IL) combined with a high resolution camera (Leica DFC290 HD) was used.

### d) Analysis of B-cell activation via CFSE-labeling.

Isolated B-cells were washed with sterile phosphate buffer saline solution (PBS). Up to 1x10⁷ cells were resuspended in 1ml protein-free PBS and incubated with CFSE (#C34554, Invitrogen/Molecular Probes) for 3 to 10 minutes at a final concentration of 2.5 µM at 37°C. CFSE loading was stopped by addition of an excess of FCS-supplemented medium. After extensive washing with FCS-containing medium, B-cells were used in co-culture experiments. Proliferation of CD19⁺ gated (B-)cells as a consequence of CFSE dilution was confirmed by flow cytometric analysis (FL-1 channel) after indicated time points.

### B-cell culture

B-cell cultures were prepared by a method similar to that described by Zubler, et al. (see e.g. Eur. J. Immunol. 14 (1984) 357-363; J. Exp. Med. 160 (1984) 1170-1183).

Briefly, single sorted B-cells were cultured in 96-well plates with 210 µl/well EL4 B5 medium with Pansorbin Cells (1:20000) (Calbiochem (Merck), Darmstadt, Deutschland), 5 % rabbit thymocyte supernatant and gamma-irradiated EL4-B5 murine thymoma cells (2×10⁴/well) for 7 days at 37 °C in an atmosphere of 5 % CO₂ in the incubator. B-cell culture supernatants were removed for screening and the cells harvested immediately for variable region gene recovery or frozen at -80 °C in 100 µl RLT buffer (Qiagen, Hilden, Germany).

Human B-cells were cultured accordingly. Human CD40L expressing feeder cells (BHK cells) were used for co-cultures and the indicated feeder mixes.

### Example 1

### Generation of the expression plasmids for full-length rabbit CD40L

The amino acid sequence for the rabbit CD40L gene was taken from GenBank, Accession number XP_002720374 (SEQ ID NO: 01).

Rabbit CD40L CDNA sequence XP_002720374 - SEQ ID NO: 01

The amino-acid sequence was back-translated into an encoding nucleic acid sequence (DNA).

### Plasmid number 7111

The gene segment encoding the synthesized rabbit CD40L cDNA was cloned into a cDNA-expression vector. In this expression vector its expression is controlled by a shortened intron A-deleted immediate early enhancer and promoter from the human cytomegalovirus (HCMV) including a human heavy chain immunoglobulin 5'-untranslated region (UTR), where the intron A with splice donor and acceptor sites are included, the full-length rabbit CD40L gene with its signal anchor sequence, and the strong polyadenylation signal from bovine growth hormone. The expression plasmid contains also an origin of replication and a B-lactamase gene from the vector pUC18 for plasmid amplification in Escherichia coli.

### Plasmid number 7112

Plasmid number 7112 was constructed in the same way as plasmid number 7111 but contains an additional hygromycin resistance gene for the generation/selection of stably transfected mammalian cell lines.

### Example 2

### Generation of rabbit CD40L expressing CHO-K1 cells

According to the manufacturer's instructions CHO-K1 (ATCC CCL-61) cells were transfected with a rabbit CD40L coding plasmid (#7112) (e.g. using lipofection method or the Nucleofector kit T (Lonza formerly Amaxa, VCA-1002) with the Nucleofector program U-17). After transfection cells were cultured in 6-well plates for 24 hours until 0.5mg/ml Hygromycin was added to establish selection pressure. Two weeks after transfection medium was supplemented with 10 % FCS (v/v). After an expansion and selection phase of five weeks the transfected cells were analyzed on a FACS Aria using a cross-reactive polyclonal goat anti-murine CD40L primary antibody (R&D Systems; AF1163) and an anti-goat IgG secondary antibody (Alexa488-labeled; Molecular Probes; #A11055). Cells with highest mean fluorescence intensity (top 5 %) were used for a single cell sort in U-bottom 96-well plates. After additional two weeks of expansion, clones were analyzed by FACS for rabbit CD40L expression.

Likewise human CD40L expressing BHK cells were obtained.

### Example 3

### Irradiation of feeder cells for co-culture

CHO clones stably expressing rabbit CD40L were grown under selection pressure (0.5 mg/ml Hygromycin) in CHO rbCD40L medium and stored in liquid nitrogen until further usage.

Two passages before using the cells as stimulators for sorted rabbit B-cells Hygromycin was removed from the medium and one day before co-culture, medium was switched to rabbit B-cell medium. Cells were harvested and adjusted to a cell concentration of 1x10⁶/ml before γ-irradiation at 50 Gy. After this, 1x10⁴ cells were plated in 100 µl per well in a U-bottom 96 well plate, centrifuged for 1 min. at 500xg and incubated overnight at 37 °C.

### Example 4

### Isolation procedure of primary antibody-secreting cells (ASCs) derived from rabbit peripheral blood -

For the isolation of rabbit B-cells whole blood of non-immunized New Zealand White rabbits was used.

In some approaches, in a first step the lymphocyte population was isolated by lympholyte density centrifugation (Biozol; #CL5120) or Ficoll Paque Plus (GE Healthcare, cat. #17-1440-03). In a second step rabbit lymphocytes were incubated with a FITC-labeled anti-rabbit IgG specific antibody (final concentration = 10 µg/ml; STAR121F; Serotec). FITC⁺ cells (rbIgG⁺ cells) were then purified via anti FITC Micro beads (#130-048-701; Miltenyi Biotec) (pool deposition).

In other approaches ("single cell deposition"), FITC⁺ cells were selected by FACS technology (see Material & Methods).

### Example 5

### Establishment of a co-culture system

In preliminary experiments stimulator cells and rabbit B-cells were mainly co-cultured at a ratio of 2:1.

Single rabbit B-cells were sorted on the plates prepared with the stimulator cells by using a FACS Aria as described in Example 4. For single B-cell co-culture, one rabbit B-cell was deposited on a monolayer of 10,000 irradiated feeder cells. If indicated a feeder-mix was added (e.g. standard supplements were 25 ng/ml IL-21, 50 U/ml IL-2, and 1:10,000 SAC) in 100 µl B-cell medium - and co-cultures were grown for 6 days at 37 °C and 5 % CO₂.

### Example 6

### Antibody production visualized by IgG-specific ELISA

After co-culture 150 µl cultivation supernatant was transferred for subsequent rabbit IgG determination. Expanded rabbit B-cells were stored in liquid nitrogen in 96-well format by adding FCS and DMSO.

Optionally, incubation length was prolonged up to 11-14 days. Therefore 150 µl freshly prepared rabbit B-cell medium with feeder mix and SAC was added and supernatant was again assessed for rabbit IgG concentration.

### Example 7

### Cultivation of IgG⁺ rabbit B-cells

Single-sorted IgG⁺ rabbit B-cells were cultured either in combination with the murine thymoma cell line EL4-B5 and rabbit thymocyte supernatant (TSN) and SAC or in the presence of a rabbit CD40L expressing CHO cell line (10,000 cells/well; +/- additional SAC) for one week. TSN was replaced by recombinant cytokines IL-2 and IL-21.

After 6 days, a rabbit IgG-specific ELISA was performed and the percentage of rabbit IgG⁺ wells (of total wells) was determined (determination of rbIgG productivity).

### Results:

The percentage of rabbit IgG⁺ wells was higher when a co-culture system comprising rabbit CD40L expressing CHO cells and a mixture of IL-2 and IL-21 was used (7.1 % vs. 6.7 % (with EL4-B5 cells)). The difference was increased if SAC was also added to the rabbit CD40L expressing CHO cells (17.9 % vs. 6.7 %). Similar results were obtained by using rabbit B-cells from different immunization campaigns against different antigens as shown in the following Table.

### Tables: B-cell co-cultures comprising different feeder mixes. Shown is the percentage of IsG⁺ wells.

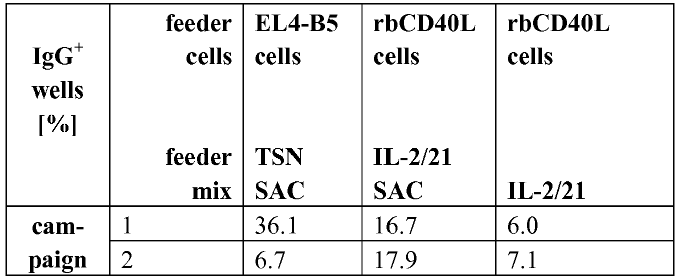

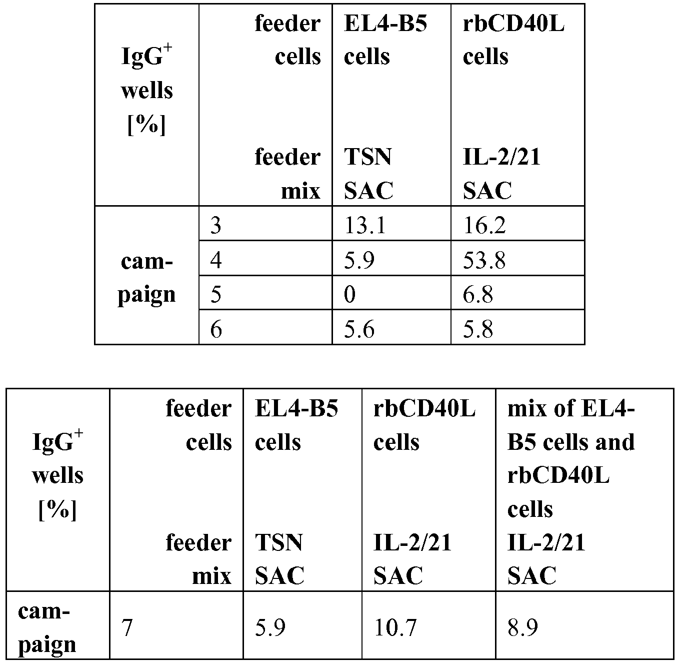

In the co-cultivation of IgG⁺CD138⁺ double-positive sorted B-cells the rbCD40L system with added cytokines and SAC did not result in IgG-producing B-cell clones. The co-culture with the EL4-B5 cells (incl. TSN and SAC) resulted in B-cell clones and IgG production as shown in the following Table (22.2 % vs. no significant proliferation observable vs. 6.0 %).

**Table: Co-cultivation of IgG⁺CD138⁺ sorted B-cells.**

| **IgG⁺ wells [%]** | **feeder cells** | **EL4-B5 cells** | **rbCD40L cells** | **rbCD40L cells** |
|---|---|---|---|---|
| | | | | |
| | **feeder mix** | **TSN SAC** | **IL-2/21 SAC** | **IL-2/21** |
| **campaign** | 8 | 22.2 | *(not detectable*/*BDL*)* | 6.0 |

| | | | | |
|---|---|---|---|---|
| *(*no significant proliferation observed)* | | | | |

### Example 8

### Synthetic rabbit B-cell culture system

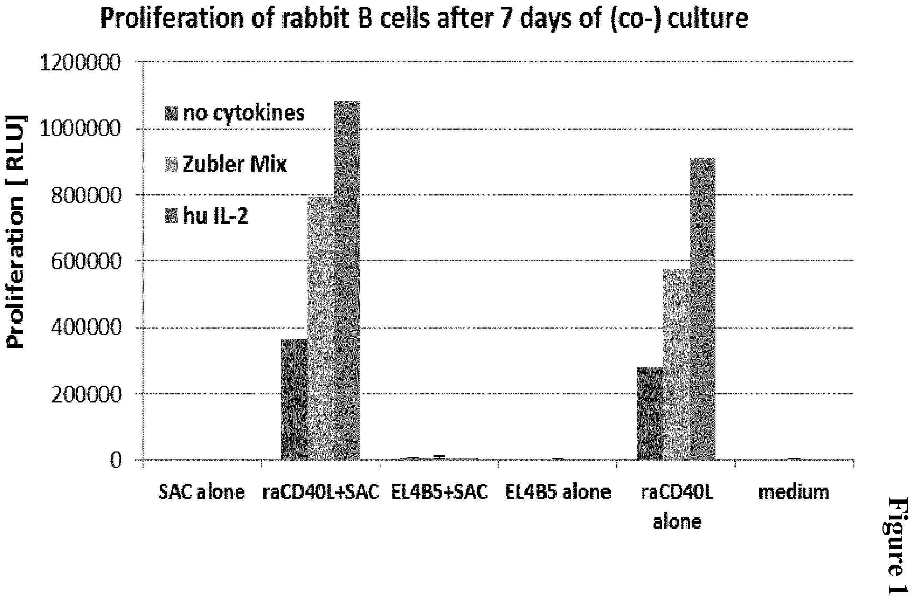

Two thousand five hundred rabbit IgG⁺ B-cells/well were co-cultivated with irradiated rabbit CD40L expressing CHO cells or EL4-B5 cells (5,000 each) without the addition of TSN. In addition to the co-cultivation without any supplements, the impact of the addition of recombinant human IL-2 vs. the Zubler-Mix was tested. After 7 days of co-cultivation the proliferation of the B-cells was determined using the CTG assay as described earlier. The co-cultivations comprising rbCD40L cells resulted in B-cell proliferation (see following Table and Figure 1). It can be seen that supplementation of IL-2 resulted in improved proliferation compared to the addition of the so called Zubler-Mix.

**Table: Proliferation of rabbit B-cells in the presence of different feeder components (medium, SAC, γ-irradiated EL4-B5 or rbCD40L expressing cells) and additional cytokines.**

| **B-cells (2,500 per well) in the presence of** | | | |
|---|---|---|---|
| | **no cytokines [RLU]** | **Zubler Mix [RLU]** | **huIL-2 [RLU]** |
| medium | 807 | 1697 | 1225 |
| SAC | 954 | 1565 | 1805 |
| EL4-B5 cells (5,000) | 1432 | 3901 | 2098 |
| EL4-B5 cells (5,000) and SAC | 8318 | 8413 | 4848 |
| rbCD40L cells (5,000) | 278750 | 577021 | 909641 |
| rbCD40L cells (5,000) and SAC | 367092 | 792172 | 1083221 |

Five hundred rabbit B-cells per well were cultured at a ratio of 1:10 with indicated feeder cells and stimuli (see Table below). After 7 days of cultivation co-cultures of rabbit B-cells with rbCD40L expressing CHO cells improved cellular growth was observed compared to cells without CD40L expression (determined via CTG assay).

**Table: B-cell proliferation [RLU/CTG assays]. 500 B-cells/well were co-cultured for 7 days with indicated cellular systems and additional recombinant cytokines/mixes.**

| **B-cells (500 per well) in** | | | | | |
|---|---|---|---|---|---|
| **the presence** | **muIL-21** | **huIL-21** | **huIL-21** | **muIL-21** | **huIL-2** |
| **of** | **huIL-2** | **huIL-2** | | | |
| | **[RLU]** | **[RLU]** | **[RLU]** | **[RLU]** | **[RLU]** |
| only B-cells | 874 | 1170 | 651 | 795 | 552 |
| SAC | 2389 | 2962 | 1314 | 1642 | 1261 |
| wt-CHO cells | 41901 | 38774 | 39538 | 37841 | 34401 |
| wt-CHO cells and SAC | 41130 | 48654 | 34626 | 33234 | 35804 |
| rbCD40L cells | 961594 | 900960 | 245470 | 326428 | 87819 |
| rbCD40L cells and SAC | 777151 | 900366 | 105349 | 158961 | 112764 |

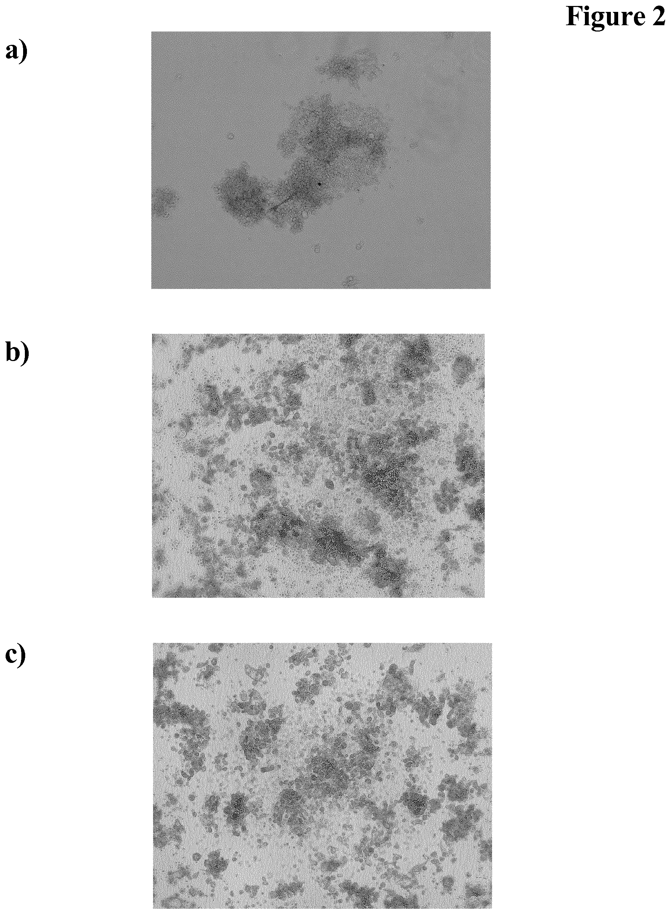

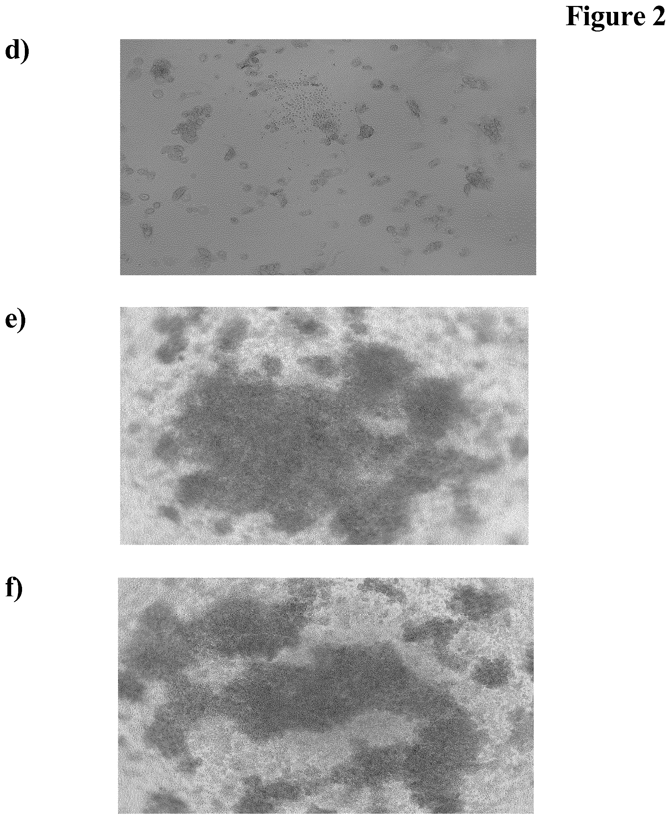

A proliferative effect was detected in the presence of rbCD40L expressing feeder cells. In principle the addition of recombinant IL-2 and IL-21 increased the cellular growth. Under these conditions, addition of SAC showed no improvement. In contrast, in other conditions addition of SAC slightly improved the proliferation. In contrast, neither IL-2, IL-21 nor the combination thereof resulted in an improvement in the EL4-B5 comprising co-culture. Exemplary wells are shown in Figure 2, where highest proliferation and cell density were obtained in the co-cultivation with rbCD40L cells (and indicated cytokines/mixes). In the same experiment, the same co-culture systems also resulted in improved rabbit IgG production after 7 days. Both the rbCD40L expressing cells and the addition of IL-21 or the combination of IL-2/21 had an effect (see Table below).

**Table: IgG production by 500 B-cells/well after 7 days [ng/ml]: IL-2 and IL-21**

| **B-cells (500 per well)** | | | | | |
|---|---|---|---|---|---|
| **Addition of** | **muIL-21** | **huIL-21** | **huIL-21** | **muIL-21** | **huIL-2** |
| **recombinant** | **huIL-2** | **huIL-2** | | | |
| **...** | **[ng/ml]** | **[ng/ml]** | **[ng/ml]** | **[ng/ml]** | **[ng/ml]** |
| **B-cells in the presence of** | | | | | |
| only B-cells | 109 | 111 | 86 | 84 | 84 |
| SAC | 111 | 111 | 84 | 84 | 84 |
| wt-CHO cells | 115 | 113 | 85 | 87 | 85 |
| wt-CHO cells and SAC | 114 | 109 | 88 | 87 | 83 |
| rbCD40L cells | 4042 | 9000 | 2727 | 2941 | 89 |
| rbCD40L cells and SAC | 4244 | 2845 | 425 | 194 | 80 |

In two separate experiments single sorted and pools of rabbit B-cells were co-cultured with EL4-B5 cells or rbCD40L expressing cells either in the presence of TSN and SAC with IL-2/IL-6/IL-21 in various combinations. In the following Table the results are shown (the number of wells with detectable rabbit IgG production was counted). The combination of rbCD40L expressing feeder cells and TSN and SAC did not result in an IgG production comparable to the EL4-B5 system. On the other hand, in the co-cultivation of rabbit B-cells with rbCD40L expressing cells and a combination of indicated cytokines such as (hu) IL-2 and IL-21 significantly more IgG was produced (shown by the percentage or number of IgG⁺ wells). No IgG⁺ wells were detected with addition of SAC alone and rbCD40L stimulator cells. SAC increased the IgG response of the IL-2/21 supplemented rbCD40L feeder system (see following Tables).

**Table: Rabbit IgG production of single sorted B-cells (percentage of rbIgG⁺ wells (left columns) or absolute numbers of IgG⁺ wells (right columns)).**

| **IgG⁺ wells [%]** | **EL4-B5 cells** | **rbCD40L cells** | **[number] IgG⁺ wells** | **EL4-B5 cells** | **rbCD40L** |
|---|---|---|---|---|---|
| TSN and SAC | 54 | 0 | TSN and SAC | 39 | 0 |
| huIL-2 and huIL-21 | 0 | 12 | huIL-2 and huIL-21 | 0 | 8 |
| huIL-2 and huIL-21 and SAC | 4 | 17 | huIL-2 and huIL-21 and SAC | 3 | 12 |
| huIL-2 and muIL-21 | 1 | 15 | huIL-2 and muIL-21 | 1 | 11 |
| huIL-2 and huIL-6 and huIL-21 | 11 | 26 | huIL-2 and huIL-6 and huIL-21 | 8 | 19 |
| huIL-2 and huIL-10 and huIL-21 | 4 | 26 | huIL-2 and huIL-10 and huIL-21 | 3 | 19 |

**Table: Rabbit IgG production of deposited B-cell pools (numbers or percentage of IgG⁺ wells).**

| | **EL4-B5 cells [number]** | **rbCD40L cells [number (%)]** |
|---|---|---|
| TSN and SAC | proliferation not detectable | 0(0%) |
| huIL-21 and huIL-2 | proliferation not detectable | 6(8.3%) |
| huIL-21 and huIL-2 and SAC | proliferation not detectable | 17 (23.6%) |
| muIL-21 and huIL-2 | proliferation not detectable | 1 (1.4%) |
| huIL-21 and huIL-2 and huIL-6 | proliferation not detectable | 0(0%) |
| huIL-21 and huIL-2 and huIL-10 | proliferation not detectable | 0(0%) |

### Example 9

### System for the generation of T cell-inhibition antibodies

After immunization with a T-cell-specific antigen a pool of antibodies is generated, which not only bind to the given antigen but may also exhibit a potential agonistic effect. In case the obtained antibody negatively interferes with T-cell biology and also interferes with the feeder system in case the culture system is based on a T-cell (like the thymoma cell line EL4-B5).

Single sorted B-cells, e.g. rabbit or human, are co-cultured either with EL4-B5 feeder cells or with CD40L expressing cells (see e.g. Example 5). The single sorted B-cells are, thus, derived from animals immunized with a T-cell antigen.

Single sorted B-cells are co-cultured with the indicated feeder mix. After about 7 days of co-cultivation the number of B-cell clones is determined and the cultivation supernatant is assessed for IgG content (as described in Materials and Methods and Example 6).

The co-cultivation can be performed with (single-sorted or pooled) B-cells derived from a human or a wild-type or a transgenic animal after immunization with antigens e.g. known for T-cell suppressive properties.

### Example 10

### Generation of a sequential B-cell culture system

Single sorted B-cells derived from an immunized mammal are co-cultured with irradiated CD40L expressing cells. In a first cultivation phase different mitogenic factors (e.g. cytokines) are added (see list below) and the co-cultivation is continued in the presence of the mitogenic factors for 1 to 14 days. After the first cultivation phase the mitogenic factor is removed from the cultivation medium (either by change of the cultivation medium for mitogenic factor free medium and/or washing of the cells or by dialysis or centrifugation/sedimentation) and the cultivation is continued in the presence of an antibody production stimulant (see list below). The IgG concentration in the cultivation supernatant is tested in the second cultivation phase after 3 or 5 or 7 or 21 days of co-cultivation.

The mitogenic stimulant can be selected from the list comprising CD40- and CD40L-interacting compounds, ICOS- and ICOS-L-interacting compounds, APRIL, BAFF, CR2, CXCL9, CXCL12 (SDF-1), CXCL13, CXCL16, Flt-3L, Interleukin-1 (α/β), Interleukin-2, Interleukin-3, Interleukin-4, Interleukin-5, Interleukin-7, Interleukin-10, Interleukin-14, Interleukin-21, SAP (signaling lymphocyte activation molecule [SLAM] associated protein), Staphylococcus A strain Cowan 1 particles (SAC; heat-killed, formalin-fixed), TLR Ligands such as LPS, different CpG ODNs or Resiquimod (R-848), TSLP, Tumor necrosis factor (TNF) alpha, type I Interferons (e.g. IFN α/β), type II interferon (e.g. IFNγ), cross-linking anti-IgG antibody, cross-linking anti-CD20 antibody, and cross-linking anti-CD27 antibody.

The antibody production stimulant can be selected from the list comprising CD40- and CD40L-interacting compounds, ICOS- and ICOS-L-interacting compounds, APRIL, BAFF, CR2, CXCL9, CXCL12 (SDF-1), CXCL13, CXCL16, Flt-3L, Interleukin-1 (α/β), Interleukin-2, Interleukin-3, Interleukin-4, Interleukin-5, Interleukin-6, Interleukin-9, Interleukin-10, Interleukin-13, Interleukin-21, Interleukin-33, SAP (signaling lymphocyte activation molecule [SLAM] associated protein), Staphylococcus A strain Cowan 1 particles (SAC; heat-killed, formalin-fixed), TLR Ligands such as LPS, different CpG ODNs or Resiquimod (R-848), TSLP, Tumor necrosis factor (TNF) alpha, type I Interferons (e.g. IFN α/β), type II interferon (e.g. IFNγ), cross-linking anti-IgG antibody, cross-linking anti-CD20 antibody, and cross-linking antiCD27 antibody.

### Example 11

### Co-cultivation system for generation of human antibodies

The effect of different cytokines in promoting human B-cell growth in the presence of human CD40L expressing BHK cells was tested as described in the examples above.

Ficoll-isolated human PBMC (2x10⁵) were seeded in a 96-well plate and cultured for 8 days in the presence of the indicated stimuli. Optionally, CD3/CD28 antibodies were added to induce a polyclonal T-cell stimulation. As can be seen from the following Table recombinant human IL-21 induced the highest human IgG production (independent from additional T-cell activation).

**Table: Human IgG production after 8 days.**

| **human PBMC plus ...** | **w/o [µg/ml]** | **CD3/CD28 [µg/ml]** |
|---|---|---|
| Medium | 0.22 | 0.09 |
| LPS | 0.00 | 0.00 |
| TNFα | 0.11 | 0.00 |
| huIL-6 | 0.16 | 0.02 |
| huIL-21 | 4.31 | 0.00 |

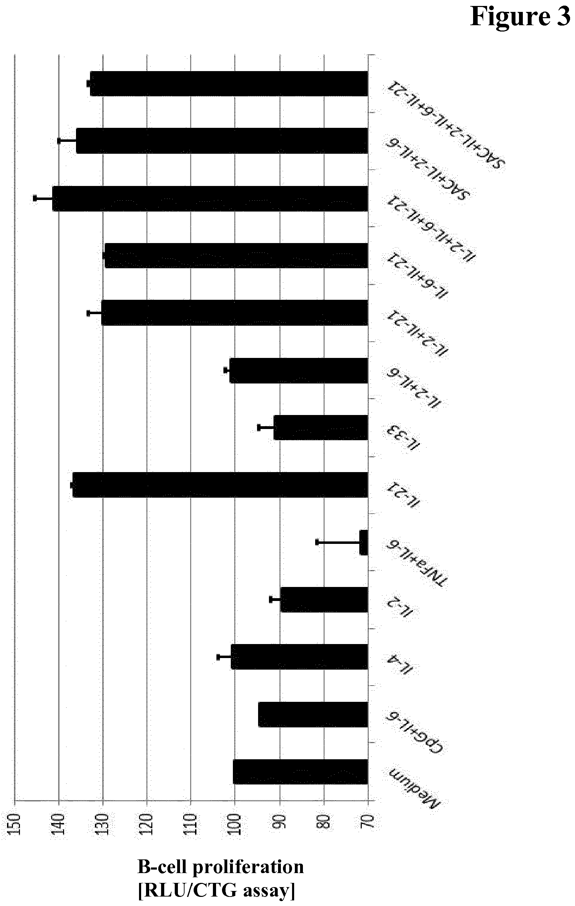

Isolated human peripheral IgG⁺ B-cells (750 per well and 2,500 per well, respectively) were co-cultured with γ-irradiated human CD40L-expressing BHK cells in the presence of different stimuli (see Table below and Figure 3).

**Table. Relative proliferation [%] of human peripheral B-cells after 8 days.**

| **human B-cells and huCD40L expressing BHK cells in the presence of ...** | **750 IgG⁺ memory B-cells/well** | **human B-cells and huCD40L expressing BHK cells in the presence of ...** | **2,500 IgG⁺ B-cells/well** |
|---|---|---|---|
| Medium | 100 | Medium | 100 |
| CpG and IL-6 | 95 | CpG and IL-6 | 113 |
| IL-4 | 101 | IL-4 | 115 |
| IL-2 | 89 | IL-2 | 101 |
| TNFα and IL-6 | 71 | TNFα and IL-6 | 101 |
| IL-21 | 136 | IL-21 | 145 |
| IL-33 | 91 | IL-33 | 105 |
| IL-2 and IL-6 | 101 | IL-2 and IL-6 | 104 |
| IL-2 and IL-21 | 130 | IL-2 and IL-21 | 150 |
| IL-6 and IL-21 | 129 | IL-6 and IL-21 | 127 |
| IL-2 and IL-6 and IL-21 | 141 | IL-2 and IL-6 and IL-21 | 152 |
| SAC and IL-2 and IL-6 | 136 | SAC and IL-2 and IL-6 | 131 |
| SAC and IL-2 and IL-6 and IL-21 | 132 | SAC and IL-2 and IL-6 and IL-21 | 101 |

It can be seen that isolated IgG⁺ memory B-cells (isolated with MACS kit # 130-094-350) as well as IgG⁺ B-cells (directly isolated via anti-IgG-microbeads (MACS kit #130-047-501) responded similar to the different stimuli. Addition of IL-21 alone (or in combination) resulted in an improved proliferation response after 8 days of co-cultivation. Co-stimulation with IL-2, IL-6 or a combination thereof did not enhance the effect neither did the addition of SAC.

The supernatants were tested for IgG production. From the following Table it can be seen that the addition of IL-21 and its combinations resulted in an improved IgG production in a co-cultivation with huCD40L expressing BHK cells.

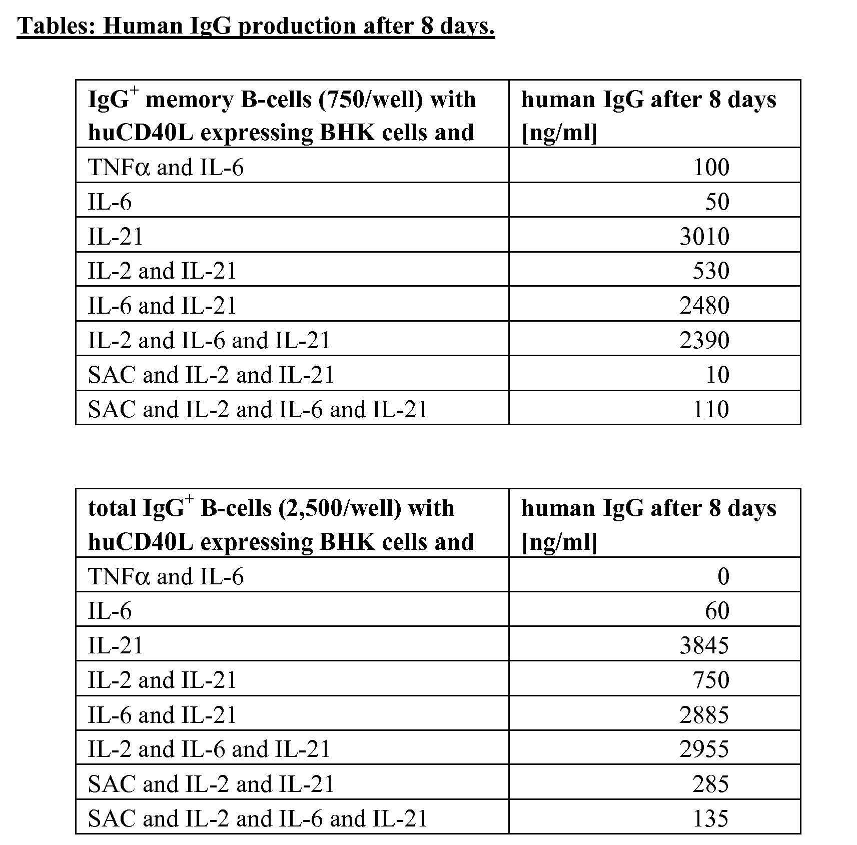

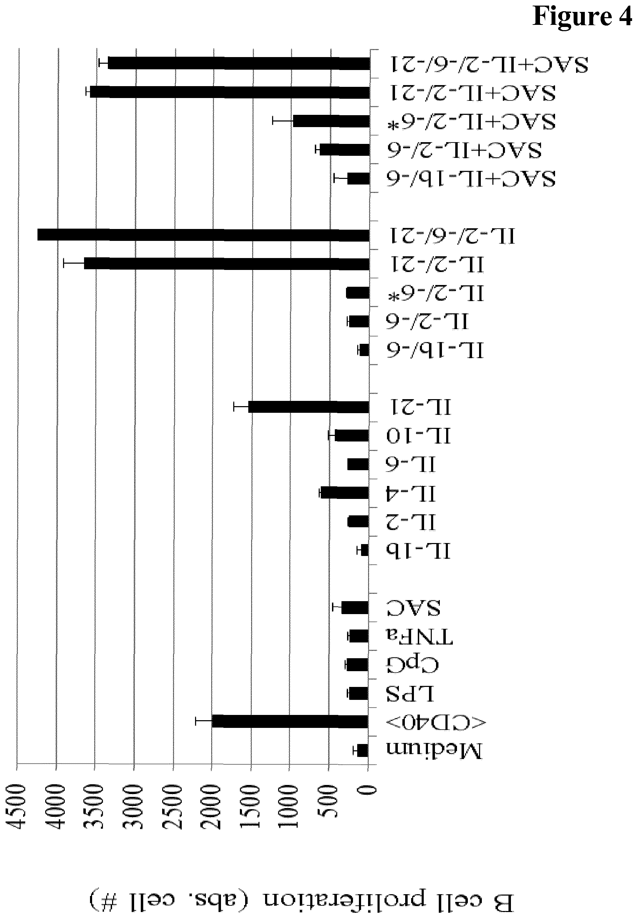

The proliferation of untouched isolated human B-cells was also assessed. B-cells were separated from PBMC using the Dynal Kit 113.13D (Invitrogen), counted and 90,000 CFSE-labeled B-cells per well were co-cultured with γ-irradiated human CD40L expressing BHK cells (CFSE-negative). The results are shown in the following Table and Figure 4.

**Table: B-cell proliferation (absolute cell numbers counted by FACS analysis of CD19⁺ cells).**

| **B**-**cells and huCD40L expressing BHK cells and** | **B-cell proliferation [cell number]** |
|---|---|
| medium | 132 |
| anti-huCD40 antibody | 1995 |
| LPS | 240 |
| CpG | 263 |
| TNFα | 241 |
| SAC | 336 |
| IL-1b | 84 |
| IL-2 | 244 |
| IL-4 | 600 |
| IL-6 | 256 |
| IL-10 | 422 |
| IL-21 | 1542 |
| IL-1b and IL-6 | 117 |
| IL-2 and IL-6 | 242 |
| IL-2 and IL-6* (IL-6 added after 48 hours) | 283 |
| IL-2 and IL-21 | 3659 |
| IL-2 and IL-6 and IL-21 | 4247 |
| SAC and IL-1b and IL-6 | 281 |
| SAC and IL-2 and IL-6 | 641 |
| SAC and IL-2 and IL-6* (IL-6 added after 48 hours) | 985 |
| SAC and IL-2 and IL-21 | 3588 |
| SAC and IL-2 and IL-6 and IL-21 | 3357 |

The absolute cell numbers are increased by the addition of IL-21 alone or IL-2/IL-21 or IL-21/IL-6/IL-21.

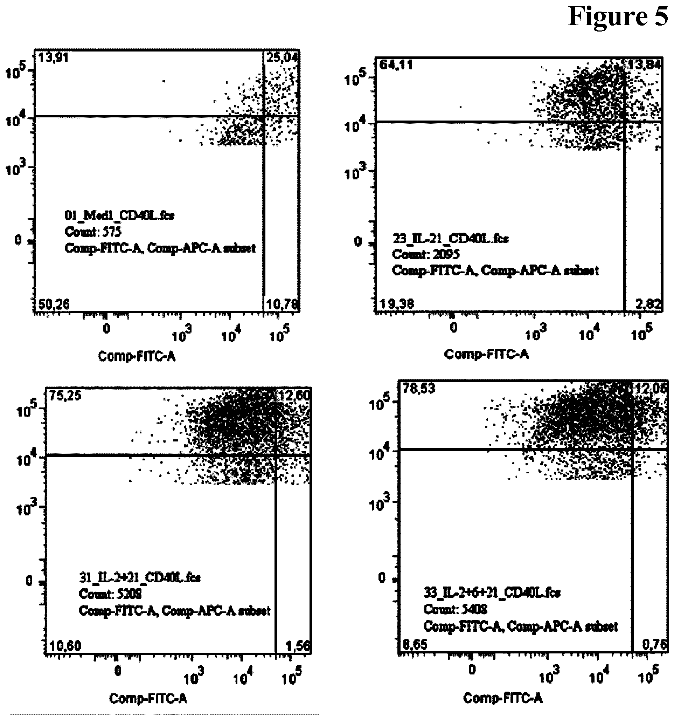

Exemplary dotplots show the CFSE-dilution as a degree of the proliferation analyzed by FACS (see Figure 5): addition of IL-21 or in combination with IL-2 and IL-2/-6 increased the proliferation of B-cells (shown are CD19⁺ gated cells).

Untouched human naive B-cells, depleted from activated B-cells, T-cells, NK-cells, monocytes, macrophages, granulocytes, platelets, plasma cells and erythrocytes (Dynal kit, Invitrogen, #113.13D) were used in co-culture with γ-irradiated human CD40L expressing BHK cells and a variety of additional stimuli. The proliferation of 50,000 initially deposited B-cells/well is shown in the Table below.

**Table: Proliferation of naive B-cells (50,000/well) in co-culture with irradiated huCD40L BHK cells for 7 days (number of proliferated B-cells detected by FACS analysis).**

| **B**-**cells and huCD40L expressing BHK cells and** | **B-cell proliferation [cell number]** |
|---|---|
| Medium | 0 |
| anti-huCD40 antibody | 62 |
| LPS | 1324 |
| CpG | 1587 |
| TNFα | 960 |
| SAC | 1147 |
| IL-1b | 2103 |
| IL-2 | 2361 |
| IL-4 | 8145 |
| IL-6 | 2619 |
| IL-10 | 4422 |
| IL-21 | 16275 |
| IL-1b and IL-6 | 989 |
| IL-2 and IL-6 | 1500 |
| IL-2 and IL-6* (IL-6 added after 48 hours) | 2265 |
| IL-2 and IL-21 | 19210 |
| IL-2 and IL-6 and IL-21 | 19017 |
| SAC and IL-1b and IL-6 | 927 |
| SAC and IL-2 and IL-6 | 2509 |
| SAC and IL-2 and IL-6* (IL-6 added after 48 hours) | 2732 |
| SAC and IL-2 and IL-21 | 19057 |
| SAC and IL-2 and IL-6 and IL-21 | 16607 |

The addition of IL-21 (either alone or in combination with IL-2 and/or IL-6) resulted in improved B-cell growth.

### Example 12

### B-cell culture system using SAC

Isolated human B-cells (5x10⁴ cells/well isolated from naive human PBMC using the negative B-cell isolation kit (#113.13D, Invitrogen)) were cultured in a 96-well round bottom plate and incubated for 6 days in the presence of the indicated supplements before proliferation (CTG assay) or human IgG production (ELISA) was determined. The results are shown in the following Table.

**Table.**

| **human B-cells** | **Proliferation** | **total huIgG in** |
|---|---|---|
| | | **supernatant** |
| **and** | **[RLU]** | **[ng/ml]** |
| - | 6453 | 0 |
| IL-2 | 22217 | 3.9 |
| SAC | 60817 | 55.1 |
| SAC and IL-6 | 69512 | 46.6 |
| SAC and IL-2 and IL-6 | 200882 | 273.9 |
| SAC and IL-2 | 295067 | 546.5 |

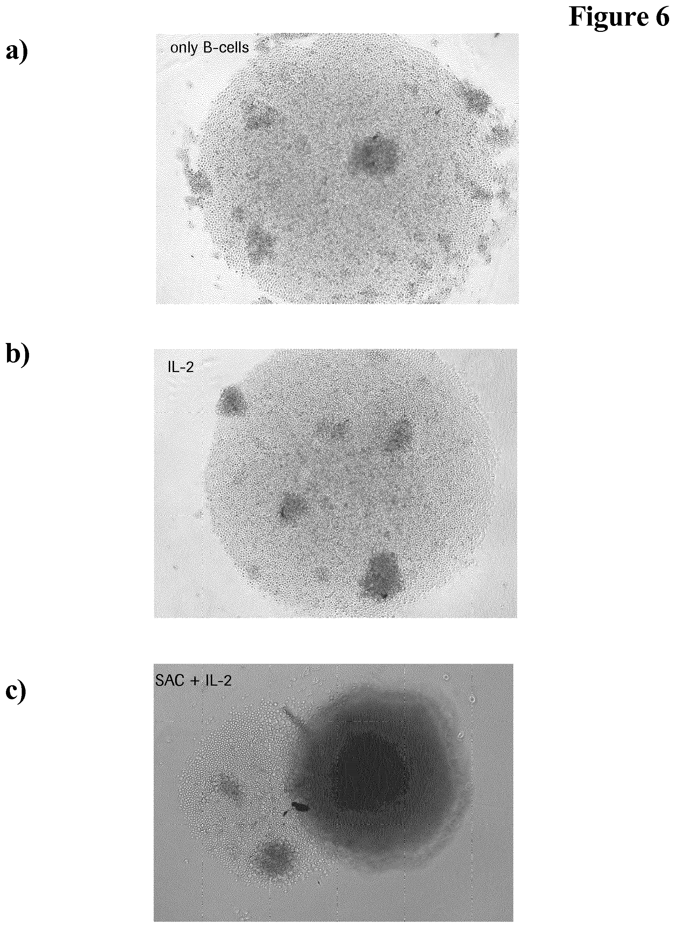

It can be seen that SAC in combination with IL-2 and to a lesser degree the combination of IL-2 and IL-6 resulted in improved B-cell proliferation and IgG production. Exemplary B-cell clusters after 3 days of incubation are shown in Figure 6.

Using a pool of purified peripheral rabbit B-cells the addition of SAC resulted in increased proliferation of B-cells (see following Tables).

**Table: Proliferation of rabbit B-cells in the presence of different feeder components (medium, SAC, γ-irradiated EL4-B5 or rbCD40L expressing cells) and additional cytokines.**

| **B**-**cells (2,500 per well) in the presence of** | | | |
|---|---|---|---|
| | **no cytokines [RLU]** | **Zubler Mix [RLU]** | **huIL-2 [RLU]** |
| medium | 807 | 1697 | 1225 |
| SAC | 954 | 1565 | 1805 |
| EL4-B5 cells (5,000) | 1432 | 3901 | 2098 |
| EL4-B5 cells (5,000) and SAC | 8318 | 8413 | 4848 |
| rbCD40L cells (5,000) | 278750 | 577021 | 909641 |
| rbCD40L cells (5,000) and SAC | 367092 | 792172 | 1083221 |

**Table: B-cell proliferation [RLU/CTG assays], 500 B-cells/well were co-cultured for 7 days with indicated cellular systems and additional recombinant cytokines/mixes.**

| **B**-**cells (500 per well) in the presence of** | | | | | |
|---|---|---|---|---|---|
| | **muIL-21** | **huIL-21** | **huIL-21** | **muIL-21** | **huIL-2** |
| | **huIL-2** | **huIL-2** | | | |
| | **[RLU]** | **[RLU]** | **[RLU]** | **[RLU]** | **[RLU]** |
| only B-cells | 874 | 1170 | 651 | 795 | 552 |
| SAC | 2389 | 2962 | 1314 | 1642 | 1261 |
| wt-CHO cells | 41901 | 38774 | 39538 | 37841 | 34401 |
| wt-CHO cells and SAC | 41130 | 48654 | 34626 | 33234 | 35804 |
| rbCD40L cells | 961594 | 900960 | 245470 | 326428 | 87819 |
| rbCD40L cells and SAC | 777151 | 900366 | 105349 | 158961 | 112764 |

The addition of SAC also resulted in an increase of the IgG production of cultivated B-cells (number of IgG positive wells and/or IgG secretion).

### Example 13

### Rabbit B-cell co-cultivation

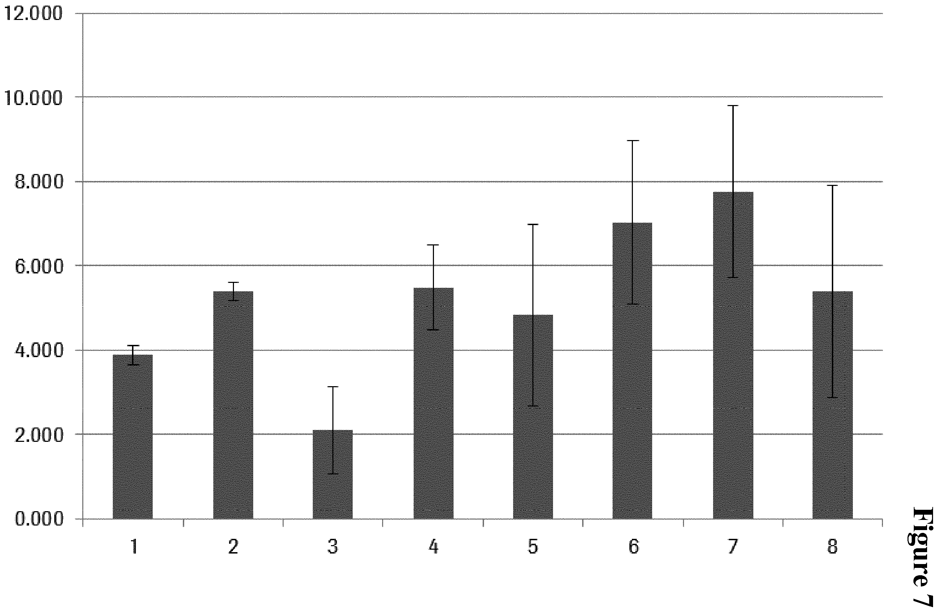

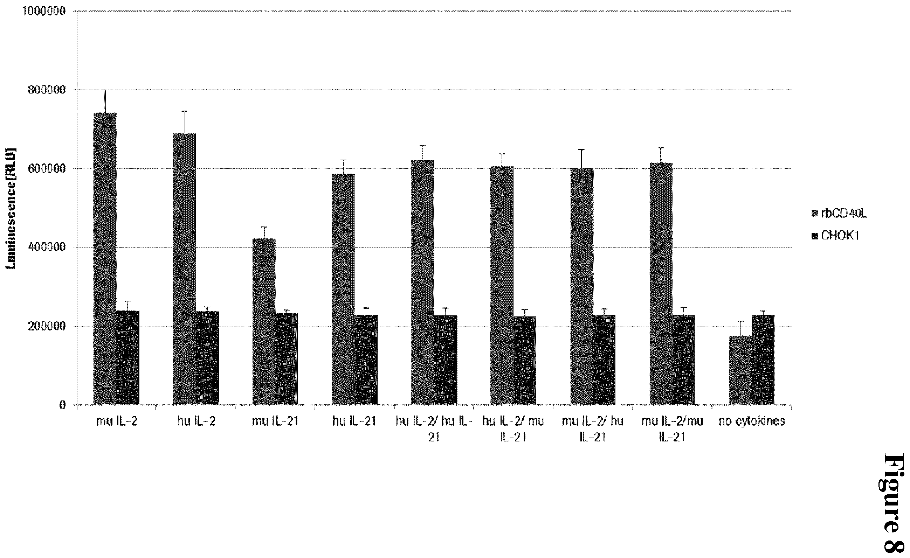

Five hundred rabbit IgG⁺ B-cells/well were co-cultivated with γ-irradiated rabbit CD40L expressing CHO cells or γ-irradiate CHO-K1 cells (10,000 each) without the addition of TSN. In addition to the co-cultivation without any supplements, the impact of the addition of recombinant human IL-2, recombinant murine IL-2, recombinant human IL-21, recombinant murine IL-21 and combinations thereof was tested. After 7 days of co-cultivation the antibody titer in the supernatant of the B-cells was determined using the ELISA as described earlier. The co-cultivations comprising rbCD40L cells in the presence of IL-2 and IL-21 resulted in B-cell proliferation and antibody secretion (see Figures 7 and 8).

### Example 14

### Rabbit B-cell co-cultivation

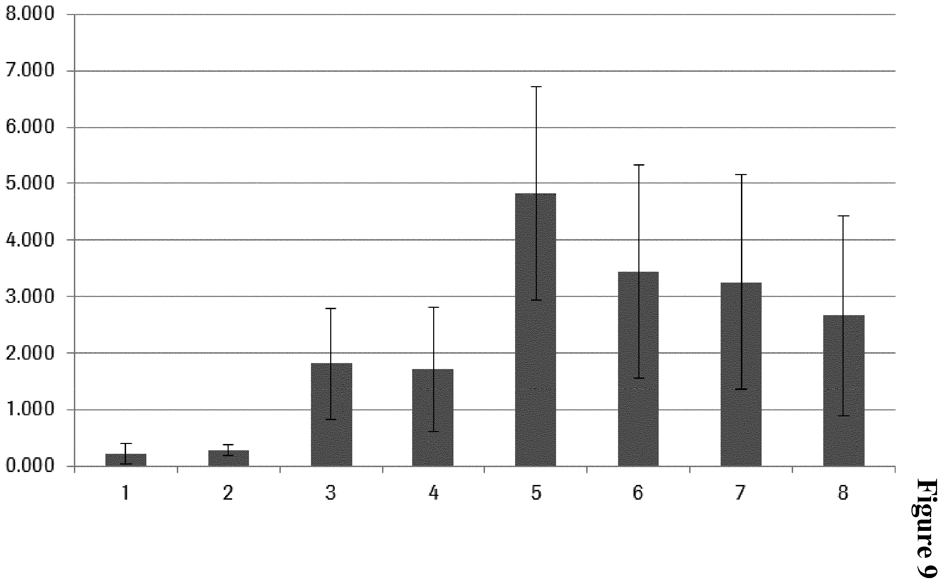

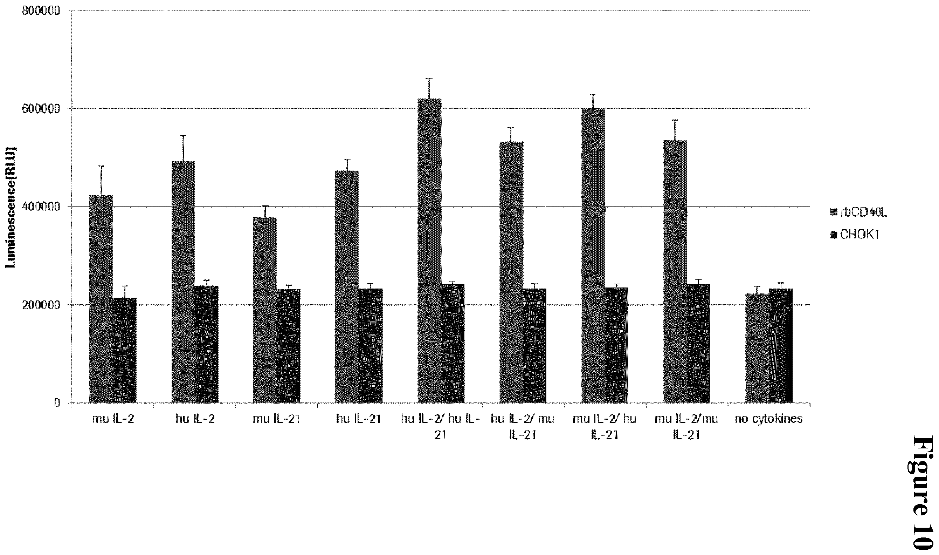

Five hundred B-cells isolated from frozen and thawed rabbit IgG⁺ PBMCs per well were co-cultivated with γ-irradiated rabbit CD40L expressing CHO cells or γ-irradiated CHO-K1 cells (10,000 each) without the addition of TSN. In addition to the co-cultivation without any supplements, the impact of the addition of recombinant human IL-2, recombinant murine IL-2, recombinant human IL-21, recombinant murine IL-21 and combinations thereof was tested. After 7 days of co-cultivation the antibody titer in the supernatant of the B-cells was determined using the ELISA as described earlier. The co-cultivations comprising rbCD40L cells in the presence of IL-2 and IL-21 resulted in B-cell proliferation and antibody secretion (see Figures 9 and 10).

### Example 15

### Synthetic rabbit B-cell culture system

### Low B-cell density:

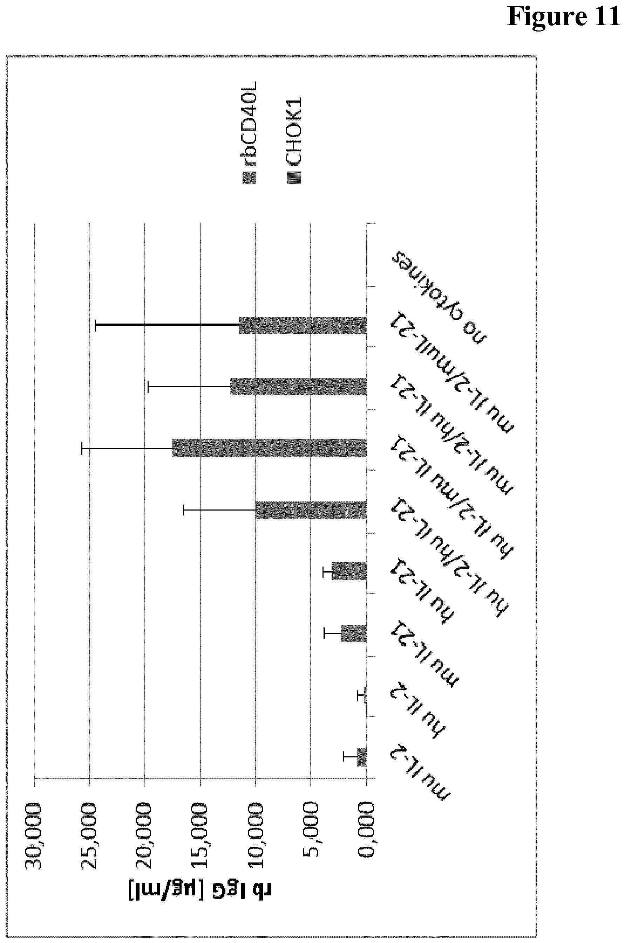

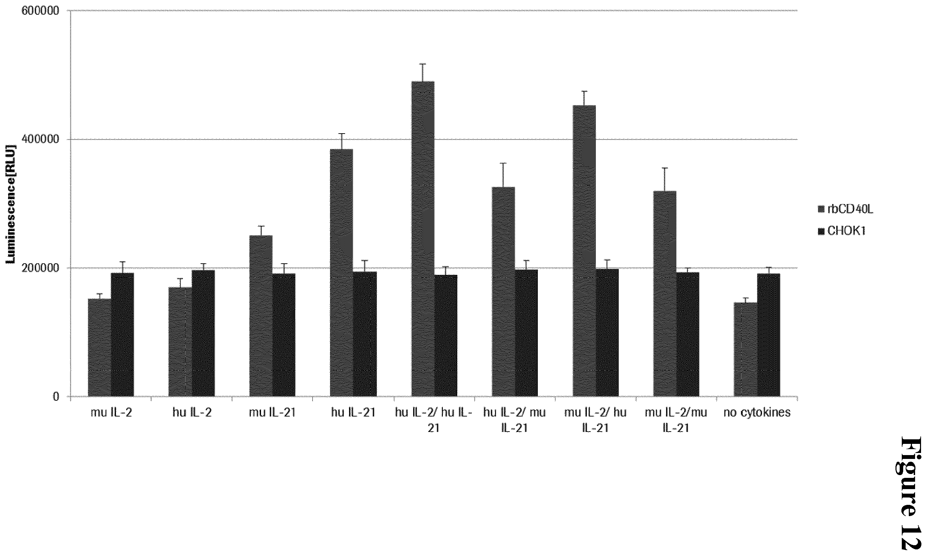

Five hundred rabbit IgG⁺ B-cells/well (isolated from an NZW rabbit) were co-cultivated with either γ-irradiated rabbit CD40L expressing CHO cells or γ-irradiated CHO-K1 cells (10,000 each) without the addition of TSN. In addition to the co-cultivation without any supplements, the impact of the addition of recombinant human IL-2, recombinant murine IL-2, recombinant human IL-21, recombinant murine IL-21 and combinations thereof was tested. After 7 days of co-cultivation the antibody titer in the supernatant of the B-cells was determined using the ELISA as described earlier. The co-cultivations comprising rbCD40L cells in the presence of IL-2 and IL-21 resulted in B-cell proliferation and antibody secretion (see Figure 11 and 12).

### High B-cell density:

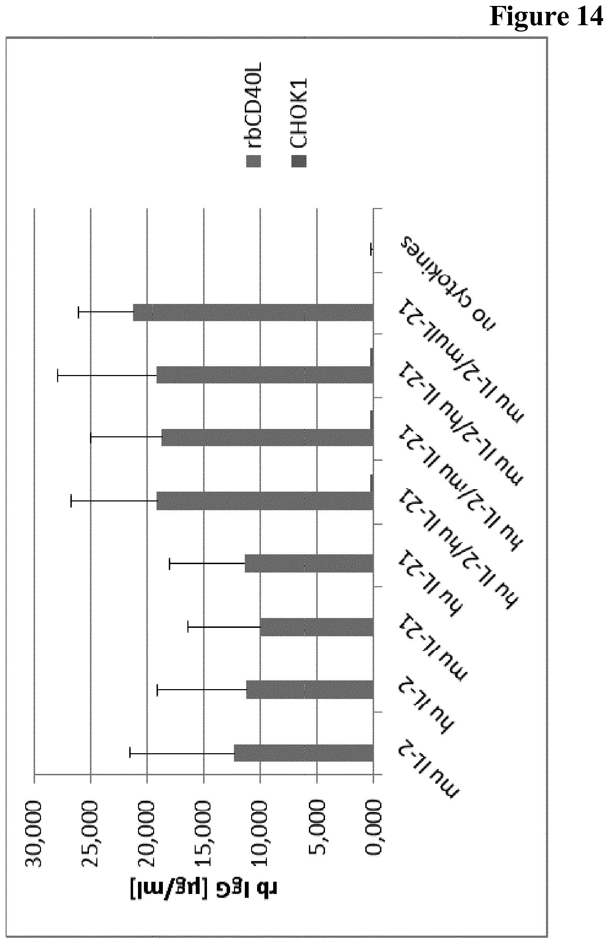

Two thousand five hundred rabbit IgG⁺ B-cells/well (from the same NZW rabbit as in the previous low density experiment) were co-cultivated with γ-irradiated rabbit CD40L expressing CHO cells or γ-irradiated CHO-K1 cells (10,000 each) without the addition of TSN. In addition to the co-cultivation without any supplements, the impact of the addition of recombinant human IL-2, recombinant murine IL-2, recombinant human IL-21, recombinant murine IL-21 and combinations thereof was tested. After 7 days of co-cultivation the antibody titer in the supernatant of the B-cells was determined using the ELISA as described earlier. The co-cultivations comprising rbCD40L cells in the presence of IL-2 and IL-21 resulted in B-cell proliferation and antibody secretion (see Figure 14).

### Example 16

### Kinetic analysis of synthetic B-cell culture system

Rabbit IgG⁺ cells are magnetically enriched from PBMC of NZW rabbit whole blood as described in Example 4. Five hundred rabbit B-cells per well are cultured at a ratio of 1:20 with γ-irradiated rabbit CD40L⁺ CHO feeder cells or control γ-irradiated CHO-K1 feeder cells (10,000 each; generation as described in examples 1-3) in the absence or presence of IL-2, IL-21 or combination of IL-2 and IL-21 (of the same or different origin, e.g. mouse and human). After 1, 2, 3, 4, 5, 6 and 7 days of cultivation (d1-d7) the co-cultures of rabbit B-cells with rabbit CD40L expressing CHO cells are assessed for rabbit IgG production in the supernatant and cellular growth. These data are compared to cells co-cultivated with feeder cells without CD40L expression and/or in the absence of additional exogenous stimuli (the same single interleukins or combination of interleukins). The growth and proliferation kinetics are determined via CTG assay and by microscopic analysis (see above).

Interleukins are applied/used in the following final concentrations:
- murine IL-2 at 50 U/ml (Roche Diagnostics GmbH, cat #11271164001)
- human IL-2 at 50 U/ml (Roche Diagnostics GmbH, cat. #11147528001)
- human IL-21 in the range of 1fold to 3fold of the ED₅₀ concentration, which varies between 10-100 ng/ml, depending on the IL-21 batch (eBioscience, cat. #14-8219)
- murine IL-21 at 100 ng/ml (R&D Systems, cat. #594-ML).

## Claims

1. A method for producing an antibody comprising the step of co-cultivating a rabbit B-cell with a rabbit CD40L expressing BHK or CHO cell in the presence of IL-2 and IL-21.

2. The method according to claim 1, **characterized in that** the B-cell is a non-mature B-cell.

3. The method according to the any one of the preceding claims, **characterized in that** the IL-2 is human IL-2 and the IL-21 is murine IL-21.

4. The method according to any one of the preceding claims, **characterized in that** the B-cell is a single deposited B-cell.

5. The method according to any one of the preceding claims, **characterized in that** the B-cells are IgG positive B-cells.

6. The method for producing an antibody according to any one of claims 1 to 5, which specifically binds to an antigen, comprising the following steps:
a) co-cultivating a pool of antibody secreting rabbit B-cells or a single antibody secreting rabbit B-cell with rabbit CD40L expressing CHO or BHK cells in the presence of IL-2 and IL-21,
b) cultivating a cell comprising a nucleic acid encoding the variable regions or a humanized variant thereof of the antibody secreted by the rabbit B-cell co-cultivated in step a) within one or more expression cassettes,
c) recovering the antibody from the cell or the cultivation medium and thereby producing an antibody, which specifically binds to an antigen.

7. The method according to claim 6, **characterized in that** the method comprises the following steps:
a) providing a population of antibody secreting rabbit B-cells preferably obtained from the blood of an experimental animal,
b) staining the cells of the population of rabbit B-cells with at least one fluorescence dye preferably with one to three, or two to three fluorescence dyes,
c) depositing single cells of the stained population of B-cells in individual containers preferably wherein the container is a well of a multi well plate,
d) cultivating the deposited individual rabbit B-cells in the presence of rabbit CD40L expressing CHO or BHK cells and IL-2 and IL21,
e) determining the binding specificity of the antibodies secreted in the cultivation of the individual rabbit B-cells,
f) determining the amino acid sequence of the variable light and heavy chain domain of specifically binding antibodies by a reverse transcriptase PCR and nucleotide sequencing, and thereby obtaining a monoclonal antibody variable light and heavy chain domain encoding nucleic acid,
g) introducing the monoclonal antibody light and heavy chain variable domain encoding nucleic acid or a variant thereof encoding a humanized version of the light and/or heavy chain variable domain in an expression cassette for the expression of an antibody,
h) introducing the nucleic acid in a cell,
i) cultivating the cell and recovering the antibody from the cell or the cell culture supernatant and thereby producing an antibody, which specifically binds to an antigen.

8. Use of a rabbit CD40L expressing BHK or CHO cell, IL-2 and IL-21 in the co-cultivation of antibody secreting rabbit B-cells for improved cell growth.

## Patentansprüche

1. Verfahren zur Herstellung eines Antikörpers umfassend den Schritt des Ko-Kultivierens einer Kaninchen-B-Zelle mit einer BHK- oder CHO-Zelle, die Kaninchen-CD40L exprimiert, in Gegenwart von IL-2 und IL-21.

2. Verfahren nach Anspruch 1, **dadurch gekennzeichnet, dass** die B-Zelle eine unreife B-Zelle ist.

3. Verfahren nach einem der vorangehenden Ansprüche, **dadurch gekennzeichnet, dass** das IL-2 humanes IL-2 ist und dass das IL-21 murines IL-21 ist.

4. Verfahren nach einem der vorangehenden Ansprüche, **dadurch gekennzeichnet, dass** die B-Zelle eine einzeln abgelegte B-Zelle ist.

5. Verfahren nach einem der vorangehenden Ansprüche, **dadurch gekennzeichnet, dass** die B-Zellen IgG-positive B-Zellen sind.

6. Verfahren zur Herstellung eines Antikörpers nach einem der Ansprüche 1 bis 5, der spezifisch an ein Antigen bindet, umfassend die folgenden Schritte:
a) Ko-Kultivieren eines Pools von Kaninchen-B-Zellen, die Antikörper sekretieren, oder einer einzelnen Kaninchen-B-Zelle, die Antikörper sekretiert, mit CHO- oder BHK-Zellen, die Kaninchen-CD40L exprimieren, in der Gegenwart von IL-2 und IL-21,
b) Kultivieren einer Zelle, die eine Nucleinsäure enthält, die die variable Regionen eines Antikörpers, der von der in Schritt a) ko-kultivierten Kaninchen-B-Zelle sekretiert wird, oder einer humanisierten Variante davon, in einer oder mehreren Expressionskassette(n) codiert,
c) Gewinnen des Antikörpers aus der Zelle oder dem Kulturmedium, und damit Herstellung eines Antikörpers, der spezifisch an ein Antigen bindet.

7. Verfahren nach Anspruch 6, **dadurch gekennzeichnet, dass** das Verfahren die folgenden Schritte umfasst:
a) Bereitstellen einer Kaninchen-B-Zell-Population, die Antikörper sekretiert, die bevorzugt aus dem Blut eines Versuchtstiers erhalten wurde,
b) Färben der Zellen der Kaninchen-B-Zellen-Population mit mindestens einem Fluoreszenzfarbstoff, bevorzugt mit einem bis drei oder zwei bis drei Fluoreszenzfarbstoffen,
c) Ablegen einzelner Zellen der gefärbten B-Zell-Population in einzelnen Behältern, wobei der Behälter bevorzugt ein Well einer Multiwellplatte ist,
d) Kultivieren der hinterlegten, individuellen Kaninchen-B-Zellen in der Gegenwart von CHO- oder BHK-Zellen, die Kaninchen-CD40L exprimieren, und IL-2 und IL-21,
e) Bestimmen der Bindespezifität der Antikörper, die beim Kultivieren der individuellen Kaninchen-B-Zellen sekretiert werden,
f) Bestimmen der Aminosäuresequenz der variablen Domänen der leichten und schweren Kette von spezifisch bindenden Antikörpern durch eine reverse Transkriptase-PCR und Nucleotidsequenzierung, und dadurch Erhalten einer Nucleinsäure, die die variable Domäne der leichten und schweren Kette eines monoclonalen Antikörpers codiert,
g) Einbringen der Nucleinsäure, die die variablen Domänen der leichten und schweren Kette des monoclonalen Antikörpers codiert, oder einer Variante davon, die die humanisierte Version der variablen Domäne der leichten und/oder schweren Kette codiert, in eine Expressionskassette für die Expression eines Antikörpers,
h) Einbringen der Nucleinsäure in eine Zelle,
i) Kultivieren der Zelle und Gewinnen des Antikörpers aus der Zelle oder dem Zellkulturüberstand und dadurch Herstellen eines Antikörpers, der spezifisch an ein Antigen bindet.

8. Verwendung einer Kaninchen-CH40L-exprimierenden CHO- oder BHK-Zelle, IL-2 und IL-21 in der Ko-Kultivierung von Kaninchen-B-Zellen, die Antikörper sekretieren, zur Verbesserung des Zellwachstums.

## Revendications

1. Procédé pour produire un anticorps, comprenant l'étape de coculture d'un lymphocyte B de lapin avec une cellule BHL ou CHO exprimant CD40L de lapin en présence de IL-2 et de IL-21.

2. Procédé suivant la revendication 1, **caractérisé en ce que** le lymphocyte B est un lymphocyte B non mature.

3. Procédé suivant l'une quelconque des revendications précédentes, **caractérisé en ce que** la IL-2 est une IL-2 humaine et la IL-21 est une IL-21 murine.

4. Procédé suivant l'une quelconque des revendications précédentes, **caractérisé en ce que** le lymphocyte B est un lymphocyte B déposé individuel.

5. Procédé suivant l'une quelconque des revendications précédentes, **caractérisé en ce que** les lymphocytes B sont des lymphocytes B positifs pour IgG.

6. Procédé pour produire un anticorps suivant l'une quelconque des revendications 1 à 5, qui se lie spécifiquement à un antigène, comprenant les étapes suivantes :
a) coculture d'un ensemble de lymphocytes B de lapin sécréteurs d'anticorps ou d'un lymphocyte B individuel de lapin sécréteur d'anticorps avec des cellules CHO ou BHK exprimant CD40L de lapin en présence de IL-2 et de IL-21,
b) culture d'une cellule comprenant un acide nucléique codant pour les régions variables ou un de leurs variants humanisés de l'anticorps sécrété par le lymphocyte B de lapin co-cultivé dans l'étape a) dans une ou plusieurs cassettes d'expression,
c) récupération de l'anticorps à partir de la cellule ou du milieu de culture et, ainsi, production d'un anticorps, qui se lie spécifiquement à un antigène.

7. Procédé suivant la revendication 6, **caractérisé en ce qu'**il comprend les étapes suivantes :
a) fourniture d'une population de lymphocytes B de lapin sécréteurs d'anticorps obtenus de préférence à partir du sang d'un animal expérimental,
b) coloration des cellules de la population de lymphocytes B de lapin avec au moins un colorant fluorescent, de préférence avec un à trois, ou deux ou trois colorants fluorescents,
c) dépôt des cellules isolées de la population colorée de lymphocytes B dans des récipients individuels, un tel récipient étant de préférence un puits d'une plaque à puits multiples,
d) culture des lymphocytes B de lapin individuels déposés en présence de cellules CHO ou BHK exprimant CD40L de lapin et de IL-2 et IL-21.
e) détermination de la spécificité de liaison des anticorps sécrétés dans la culture des lymphocytes B individuels de lapin,
f) détermination des séquences d'aminoacides des domaines de chaînes légères et de chaînes lourdes variables d'anticorps à liaison spécifique par une PCR avec transcriptase inverse et par séquençage de nucléotides et, ainsi, obtention d'un acide nucléique codant pour les domaines de chaînes légères et de chaînes lourdes variables d'anticorps monoclonal,
g) introduction de l'acide nucléique codant pour les domaines variables de chaînes légères et de chaînes lourdes d'anticorps monoclonal ou d'un de ses variants codant pour une version humanisée des domaines variables de chaînes légères et/ou de chaînes lourdes dans une cassette d'expression pour l'expression d'un anticorps,
h) introduction de l'acide nucléique dans une cellule,
i) culture de la cellule et récupération de l'anticorps à partir de la cellule ou du surnageant de culture cellulaire et, ainsi, production d'un anticorps qui se lie spécifiquement à un antigène.

8. Utilisation d'une cellule BHK ou CHO exprimant CD40L de lapin, de IL-2 et de IL-21 dans la coculture de lymphocytes B de lapin sécréteurs d'anticorps pour une croissance cellulaire améliorée.
